# EUROPEAN PATENT APPLICATION

(11) **EP 4 725 459 A2**
(43) Date of publication of application: **15.04.2026**
(21) Application number: 26154092.6
(22) Date of filing: 10.07.2024
(51) Int. Cl.: A61F 2/915

(54) **MULTI-DIRECTIONAL HINGING STENT WITH ONE OR MORE FUNNEL SHAPED CONNECTORS**

(30) Priority: 10.07.2023 US 202363512887 P
(62) Divisional of application: 24749080.8
(71) Applicant: Atrium Medical Corporation, Merrimack, NH 03054 (US)
(72) Inventor: LABRECQUE, Roger, Merrimack, NH 03054 (US); HEIM, David, Merrimack, NH 03054 (US)
(74) Representative: EIP

(57) **Abstract**

An expandable multi-directional hinging stent radially expands from compressed to expanded configurations. The stent includes radially expandable rings aligned in series along a common axis and defining a common lumen of the stent extending through the rings; and elongated connectors extending between adjacent ones of the rings. A first one of the connectors and a second one of the connectors are connected to and extend between a first one of the rings and an immediately adjacent second one of the rings. A shortest distance about a circumference of the stent between a connection point between the first connector and the first ring and a connection point between the second connector and the first ring is less than a shortest distance about a circumference of the stent between a connection point between the first connector and the second ring and the second connector and the second ring.

## Description

### PRIORITY CLAIM

The present disclosure claims priority to U.S. Provisional Patent Application Serial No. 63/512,887 filed July 10, 2023; the disclosure of which is incorporated herewith by reference.

### BACKGROUND OF THE DISCLOSURE

### Field of the Disclosure

The present disclosure relates to expandable, intraluminal devices for use within a body passageway or duct and, more particularly, to implantable stents comprising one or more elongated connectors exhibiting a funnel shape to configure the implantable stent to hinge in one or multiple predetermined directions with a relatively reduced amount of force.

### Description of Related Art

A common method for treating stenosed or aneuryzed vessels or other blocked passageways is with an expandable prosthesis, such as a stent, which is configured to be deployed in the vessel or passageway in an expanded configuration to maintain patency or continuity of the vessel or passageway. Stents may also be used as fixation devices for anchoring a medical device within a vessel or passageway. Stents can be bare, coated, or covered. The cover can be constructed from a biocompatible material, such as polytetrafluoroethylene (PTFE) or expanded polytetrafluoroethylene (ePFTE). An exemplary covered stainless steel stent is the iCAST/V12 balloon expandable stent manufactured by Getinge AB.

In many surgical procedures, a stent is configured to be delivered to a target site, expanded, and affixed in place. For example, in a fenestrated endovascular aneurysm repair (FEVAR) procedure, a number of stents may be placed within pre-formed openings or fenestrations in a main body implant or endoprosthesis to create a connection between the main body implant and target branch vessels or conduits. In vascular applications, one or more covered stents can protrude into an aortic main body implant or endoprosthesis for a few millimeters. Once deployed and affixed in place, the one or more stents create an enclosed lumen space for passage of blood from the main body implant or endoprosthesis to the target vessels. The stents can also provide increased reinforcement of the vessel wall, in order to maintain a cleared lumen or passageway.

For certain target sites and surgical procedures, the deployed stent is required to adopt a bent, curved, hinged, or twisted configuration to conform to a shape of a particular vessel, passageway, or opening between adjacent vessels or passageways. It is important that the stent is capable of adopting the bent, curved, hinged, or twisted configuration without kinking or deforming to such a degree as to restrict fluid flow through a lumen of the stent. In some instances, stents can be formed from flexible materials so that the stent can be easily bent to any desired configuration. However, stents formed from flexible materials may not be strong enough to reinforce walls of a vessel or passageway or to resist collapsing when exposed to normal anatomical forces. As such, stents formed partially or entirely from highly flexible materials may not maintain patency or continuity of fluid flow through the passageway or vessel when deployed at a target site and may not be suitable for use in certain surgical procedures. For example, stents formed from flexible materials may have a relatively low radial stiffness and/or axial stiffness.

Stents formed from highly flexible materials also may not be suitable for delivery using a balloon expandable catheter. Balloon expandable stents are configured to be crimped to a balloon of a balloon expandable catheter. During delivery, the balloon expandable stent should maintain adequate retention to the catheter so as not to become dislodged until expanded at the target site. Particularly, balloon expandable stents should demonstrate the ability to be delivered and positioned accurately without extensive foreshortening or "watermelon seeding," where the stent unexpectedly slides off of the distal end of the balloon. The stent should also be designed with predictable recoil properties after deployment to maintain proper wall apposition. For example, stents that are placed in fenestrations must be strong enough to resist migration of the associated endo-graft and not collapse due to shear forces. Stents formed from highly flexible materials may not be capable of remaining in place when crimped to a balloon catheter or when deployed within a fenestration.

Many different stent designs are known to those skilled in the art. Known stent designs can include combinations of different types of framing structures, such as helical coils, meshes, lattices, or interconnected rings. Such framing structures can be made from, for example, stainless steel and/or cobalt chromium. In one common design, a stent can include a series of cylindrical rings aligned in a series along a central longitudinal or common axis. The rings can be fixedly secured to one another by a plurality of elongated connectors, such as longitudinally extending struts. Stents formed from rings and elongated connectors may have sufficient radial strength to support a vessel wall but may not be sufficiently flexible to be deployed in a curved or bent configuration. Some stent designs use bent connectors, struts, or pigtail elongated connectors to provide added flexibility. There are also designs where connectors attach via a loose press fit, so that connectors have more freedom of movement. In other examples, stent designs include drill holes in the stent struts to make them more flexible.

However, there is a need for alternative stent designs that are sufficiently flexible to avoid kinking and/or to adopt certain bent, curved, hinged, or twisted configurations, while remaining sufficiently rigid to be crimped to a balloon catheter, support or reinforce a vessel or passageway, and resist migration from a target site or deployment location. In particular, there is a need for stent designs that are flexible enough for use with balloon catheters and which do not kink, while maintaining sufficient column strength to address problems caused by foreshortening and to maintain consistent stent length.

### SUMMARY OF THE DISCLOSURE

According to an aspect of the disclosure, an expandable multi-directional hinging stent configured to radially expand from a compressed configuration to an expanded configuration includes a plurality of radially expandable rings aligned in series along a common axis and defining a common lumen of the stent extending through the plurality of rings. The stent also includes a plurality of elongated connectors extending between rings of the plurality of rings. The plurality of elongated connectors includes at least a first connector and a second connector connected to and extending between a particular ring and an immediately adjacent ring of the plurality of rings. A shortest distance about a circumference of the stent between connection points between the first connector and the second connector and the particular ring is less than a shortest distance about a circumference of the stent between connection points between the first connector and the second connector and the immediately adjacent ring.

According to another aspect of the disclosure, an expandable multi-directional hinging stent configured to radially expand from a compressed configuration to an expandable configuration includes a first radially expandable ring and a second radially expandable ring. The first and second rings are aligned in series along a common axis and define a common lumen of the stent extending through the first and second rings. The stent also includes a set of elongated connectors comprising a first pair of the elongated connectors and a second pair of the elongated connectors. Each of the elongated connectors of the first pair and the second pair comprises at least one angled segment extending between the first ring and the second ring forming a substantially funnel shape. The first pair of elongated connectors and the second pair of the elongated connectors create a hinging axis extending through the expandable multi-directional hinging stent. The expandable multi-directional hinging stent is configured to hinge about the hinging axis at the first and second rings when subject to a force that is non-parallel to the common axis. The force to hinge the expandable multi-directional hinging stent about the hinging axis is less than a further force required to hinge the expandable multi-directional hinging stent other than about the hinging axis at the first and second radially expandable rings.

According to another aspect of the disclosure, an expandable multi-directional hinging stent configured to radially expand from a compressed configuration to an expanded configuration includes a multi-directional hinging flexible portion. The flexible portion includes a plurality of radially expandable rings aligned in series along a common axis and defining a common lumen of the stent extending through the plurality of rings. The flexible portion includes a plurality of elongated connectors extending between rings of the plurality of rings. Pairs of the elongated connectors form a substantially funnel shape. A set of the pairs of the elongated connectors is arranged to form a hinging axis between a particular ring of the plurality of rings and an immediately adjacent ring of the plurality of rings. The elongated connectors are arranged to form at least two funneled hinges between a particular ring of the plurality of rings and an immediately adjacent ring of the plurality of rings. The stent also includes a rigid portion connected to and aligned in series along the common axis with the multi-directional hinging flexible portion. The rigid portion is configured to resist bending forces applied thereto such that a first bending force required to bend the rigid portion by a predetermined distance is greater than a second bending force required to bend the flexible portion about the hinging axis.

Non-limiting illustrative examples of embodiments of the present disclosure will now be described in the following numbered clauses:
Clause 1: An expandable multi-directional hinging stent configured to radially expand from a compressed configuration to an expanded configuration, the expandable stent comprising: a plurality of radially expandable rings aligned in series along a common axis and defining a common lumen of the expandable stent extending through the rings; and a plurality of elongated connectors extending between adjacent ones of the rings, wherein a first one of the elongated connectors and a second one of the elongated connectors are connected to and extend between a first one of the rings and an immediately adjacent second one of the rings, and wherein a shortest distance about a circumference of the expandable stent between a connection point between the first connector and the first ring and a connection point between the second connector and the first ring is less than a shortest distance about a circumference of the expandable stent between a connection point between the first connector and the second ring and the second connector and the second ring.
Clause 2: The expandable stent of clause 1, wherein each of the rings comprises multiple linear segments connected together end-to-end forming peaks and valleys around a circumference of the ring.
Clause 3: The expandable stent of clause 2, wherein the connection point between the first connector and the first ring and the connection point between the second connector and the first ring are on adjacent linear segments separated from one another by a valley, and/or wherein the connection point between the first connector and the second ring and the connection point between the second connector and the second ring are on non-adjacent linear segments.
Clause 4: The expandable stent of clause 3, wherein the non-adjacent linear segments of the second ring are separated from one another by a ring length comprising at least two complete linear segments connected through a valley of the second ring.
Clause 5: The expandable stent of clause 1, wherein each of the elongated connectors comprises two axially extending segments and an angled segment between the two axially extending segments angled relative to the common axis of the expandable stent, wherein the angled segment of the first connector is angled in a first direction relative to the common axis of the expandable stent and the angled segment of the second connector is angled in a second direction relative to the common axis of the expandable stent, the second direction being different from the first direction.
Clause 6: The expandable stent of clause 1, wherein the elongated connectors are arranged along a spiral extending axially through the rings.
Clause 7: The expandable stent of clause 1, wherein the first connector is symmetrical to the second connector about a line parallel to the common axis of the expandable stent.
Clause 8: The expandable stent of clause 1, wherein the first connector and the second connector form a first group, wherein the elongated connectors further comprise a second group comprising a third connector and a fourth connector extending between the first ring and the second ring.
Clause 9: The expandable stent of clause 8, wherein the first group and the second group are circumferentially offset about the first ring and the second adjacent ring such that a hinging axis is created that extends through the expandable stent based on a positioning of the first group and the second group, the expandable stent configured to hinge about the hinging axis between the first ring and the second ring.
Clause 10: The expandable stent of clause 9, wherein the first group and the second group are circumferentially offset from one another by 180 degrees.
Clause 11: The expandable stent of clause 9, wherein the expandable stent is configured to hinge between the first and second rings about the hinging axis with a first amount of force reduced relative to a second amount of force required to hinge the expandable stent between the first and second rings about an axis other than the hinging axis.
Clause 12: The expandable stent of clause 9, wherein the plurality of elongated connectors prevents kinking when the expandable stent hinges about the hinging axis.
Clause 13: The expandable stent of clause 8, wherein the first connector and the second connector are paired to exhibit a funnel shape, the funnel shape being oriented in one of a first direction with a narrow hinge point closer to a proximal end of the expandable stent and a wide base closer to a distal end of the expandable stent or a second direction with the narrow hinge point closer to the distal end of the expandable stent and the wide base closer to the proximal end of the expandable stent.
Clause 14: The expandable stent of clause 13, wherein, between the first ring and the second ring, the funnel shape of the first group and the second group is one of both facing the first direction, both facing the second direction, or one facing the first direction and the other facing the second direction.
Clause 15: The expandable stent of clause 13, wherein, at progressive pairs of the radially expandable rings, the funnel shape of the first group and the second group is facing one of the first or second direction or alternates between the first and second directions.
Clause 16: The expandable stent of clause 1, wherein the rings comprise: the first ring, the second ring, and a third radially expandable ring, wherein the first ring, the second ring, and the third ring are aligned in series along the common axis of the expandable stent, and wherein the elongated connectors comprise a third connector and a fourth connector axially offset from the first connector and the second connector the third connector and the fourth connector extending between the second ring and the third ring.
Clause 17: The expandable stent of clause 16, wherein the first connector and the second connector are circumferentially offset from the third connector and the fourth connector by no more than 180 degrees.
Clause 18: The expandable stent of clause 16, wherein the expandable stent is configured to bend about a hinging axis between the first ring and the second ring with a first amount of force in a first direction and to bend about a further hinging axis created by the third and fourth connectors between the second ring and the third ring with the first amount of force in a second direction different than the first direction.
Clause 19: An expandable multi-directional hinging stent configured to radially expand from a compressed configuration to an expandable configuration, the expandable stent comprising: a first radially expandable ring; a second radially expandable ring, the first and second rings being aligned in series along a common axis immediately adjacent to one another and defining a common lumen of the expandable stent extending through the first and second rings; and a set of elongated connectors comprising a first pair of the elongated connectors and a second pair of the elongated connectors, each of the elongated connectors of the first pair and the second pair comprising at least one angled segment extending between the first ring and the second ring forming a substantially funnel shape, wherein the first pair of elongated connectors and the second pair of the elongated connectors create a hinging axis extending through the expandable stent, the expandable stent configured to hinge between the first and second rings about the hinging axis at the first and second rings when subject to a force that is non-parallel to the common axis, and wherein the force to hinge the expandable stent about the hinging axis is less than a further force required to hinge the expandable stent between the first and second rings about an axis other than the hinging axis.
Clause 20: An expandable multi-directional hinging stent configured to radially expand from a compressed configuration to an expanded configuration, the expandable stent comprising: a multi-directional hinging flexible portion comprising: a plurality of radially expandable rings aligned in series along a common axis and defining a common lumen of the expandable stent extending through the rings, and a plurality of elongated connectors connecting immediately adjacent ones of the rings, a first pair of the elongated connectors extending between a first one of the rings and a second one of the rings immediately adjacent to the first ring forming a substantially funnel shape and a second pair of the elongated connectors extending between the first ring and the second ring forming a substantially funnel shape, the first and second pairs of the elongated connectors being arranged to form a hinging axis between the first and second rings; and a rigid portion connected to and aligned in series along the common axis with the flexible portion, the rigid portion being configured to resist bending forces applied thereto such that a first bending force required to bend the rigid portion by a predetermined distance is greater than a second bending force required to bend the flexible portion about the hinging axis.

An expandable multi-directional hinging stent configured to radially expand from a compressed configuration to an expanded configuration, the expandable stent comprising: a multi-directional hinging flexible portion comprising: a plurality of radially expandable rings aligned in series along a common axis and defining a common lumen of the stent extending through the plurality of rings, and a plurality of elongated connectors extending between rings of the plurality of rings, pairs of the elongated connectors forming a substantially funnel shape, a set of the pairs of the elongated connectors arranged to form a hinging axis between a particular ring of the plurality of rings and an immediately adjacent ring of the plurality of rings; and a rigid portion connected to and aligned in series along the common axis with the multi-directional hinging flexible portion, the rigid portion being configured to resist bending forces applied thereto such that a first bending force required to bend the rigid portion by a predetermined distance is greater than a second bending force required to bend the flexible portion about the hinging axis.

These and other features and characteristics of the invention, as well as the methods of operation and functions of the related elements of structures and the combination of parts and economies of manufacture, will become more apparent upon consideration of the following description and appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and the claims, the singular form of "a", "an", and "the" include plural referents unless the context clearly dictates otherwise.

### BRIEF DESCRIPTION OF THE DRAWINGS

FIG. 1A is a front view of a stent in an expanded configuration, according to an aspect of the disclosure;
FIG. 1B is a flattened view of a stent including both a flexible portion and a rigid portion, according to an aspect of the disclosure;
FIG. 1C is a front view of a covered stent in an expanded configuration, according to an aspect of the disclosure;
FIG. 1D is a flattened view of a stent including a rigid portion formed by axial struts extending between rings of the stent, according to an aspect of the disclosure;
FIG. 2A is a flattened view of a flexible portion of a stent, according to an aspect of the disclosure;
FIG. 2B is an enlarged view of the flexible portion of FIG. 2A showing elongated connectors exhibiting a funnel shape;
FIG. 2C is a perspective view of a stent including a flexible portion and a rigid portion, according to an aspect of the disclosure;
FIGS. 3A and 3B are flattened views of flexible portions of other exemplary stents, according to other aspects of the disclosure;
FIGS. 4A, 4B, and 4C are flattened views of flexible portions of other exemplary stents, according to other aspects of the disclosure;
FIG. 5 is a flow chart showing a method for deploying a stent, according to an aspect of the disclosure;
FIG. 6 is a graph comparing a bending response for different conventional and multi-directional hinging stents, according to aspects of the disclosure;
FIG. 7A is a front view of a stent including the flexible portion of FIG. 2A expanded to a target diameter in a straight orientation, according to an aspect of the disclosure;
FIG. 7B is a front view of the stent of FIG. 7A in a bent orientation, according to an aspect of the disclosure;
FIG. 8 is a graph comparing axial compression force and displacement for different conventional and multi-directional hinging stents, according to aspects of the present disclosure;
FIG. 9A is a front view showing a conventional rigid stent about a cylindrical object in an expanded configuration;
FIG. 9B is a front view of the stent of FIG. 9A in an axially compressed configuration;
FIG. 9C is a front view showing a multi-directional hinging stent about a cylindrical object in an expanded configuration, according to an aspect of the present disclosure;
FIG. 9D is a front view of the stent of FIG. 9C in an axially compressed configuration;
FIG. 9E is a front view showing a multi-directional hinging stent including elongated connectors exhibiting a funnel shape about a cylindrical object in an expanded configuration, according to an aspect of the present disclosure;
FIG. 9F is a front view of the stent of FIG. 9E in an axially compressed configuration;
FIG. 10A is a computer generated representation showing a conventional stent bent in a cantilever test; and
FIG. 10B is a computer generated representation showing a flexible portion of a multi-directional hinging stent including features of the present disclosure bent in a cantilever test.

### DESCRIPTION OF THE INVENTION

The illustrations generally show illustrative and non-limiting aspects of the devices, assemblies, and methods of the present disclosure. While the descriptions present various aspects of the devices and assemblies, it should not be interpreted in any way as limiting the disclosure. Furthermore, modifications, concepts, and applications of the disclosure's aspects are to be interpreted by those skilled in the art as being encompassed by, but not limited to, the illustrations and descriptions herein.

Further, for purposes of the description hereinafter, the terms "end", "upper", "lower", "right", "left", "vertical", "horizontal", "top", "bottom", "lateral", "longitudinal", "radial", and derivatives thereof shall relate to the disclosure as it is oriented in the drawing figures. The term "proximal" refers to an end of the device that is configured to be manipulated by a user or, for an implanted device, the side or end of the device that remains closest to the implantation site, when the device is deployed. The term "distal" refers to the end of the device opposite from the proximal end, which can be the end of the device farthest away from portions of the device intended to be manipulated be a user. For an implanted device, the "distal" end of the device is the end of the device implanted farthest into vasculature of a patient and/or that is farthest away from the implantation site. However, it is to be understood that the disclosure may assume various alternative variations and step sequences, except where expressly specified to the contrary. It is also to be understood that the specific devices and processes illustrated in the attached drawings, and described in the following specification, are simply exemplary aspects of the disclosure. Hence, specific dimensions and other physical characteristics related to the aspects disclosed herein are not to be considered as limiting. For the purpose of facilitating understanding of the disclosure, the accompanying drawings and description illustrate preferred aspects thereof, from which the disclosure, various aspects of its structures, construction and method of operation, and many advantages may be understood and appreciated.

With reference to the figures, the present disclosure is directed to expandable stents 10, 110 with a multi-directional hinging mechanism that allows the stents 10, 110 to bend or flex in one or multiple directions and/or that allows different portions of the stents 10, 110 to bend or flex in different directions. The stents 10, 110 of the present disclosure can be radially expanded using, for example, an expandable catheter, such as a dilating or balloon catheter. While expandable stents are often used for endovascular procedures, such as Fenestrated Endovascular Aortic Repair (FEVAR), Branched Endovascular Aortic Repair (BEVAR), chimney Endovascular Aortic Repair (chEVAR), peripheral, coronary, bilary, or other stenting procedures, it is understood that arrangements of the stents 10, 110 disclosed herein are not limited to use with endovascular procedures. For example, the stent designs disclosed herein can be adapted for use in any number of medical applications and procedures in which a stenting structure could be used to maintain fluid flow through a body lumen and/or for positioning of a medical device within a body lumen. In particular, medical devices including implantable grafts, drug delivery devices, filters, shunts, and similar medical devices could all be modified to include the arrangements of expandable rings, elongated connectors, and/or struts of the present disclosure. Furthermore, stent designs of the present disclosure may be deployed in other structures besides blood vessels, such as the small intestine, large intestine, biliary ducts, or any other anatomical site.

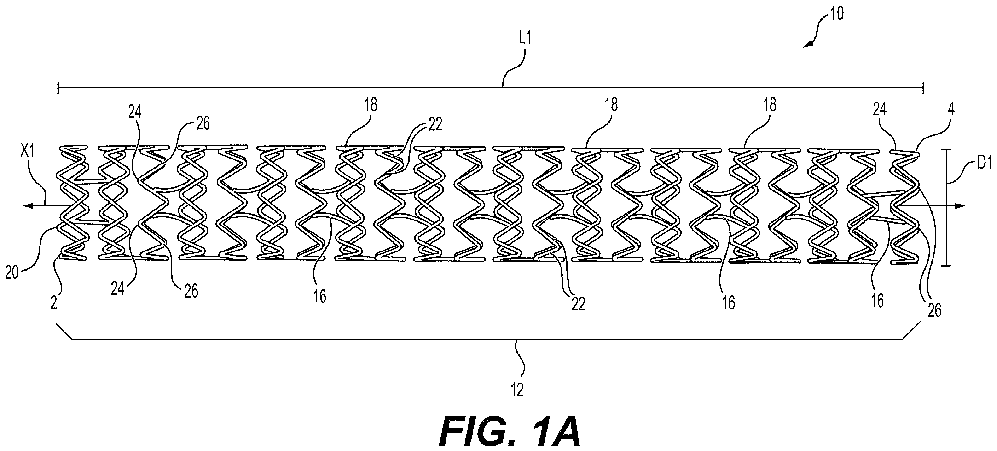

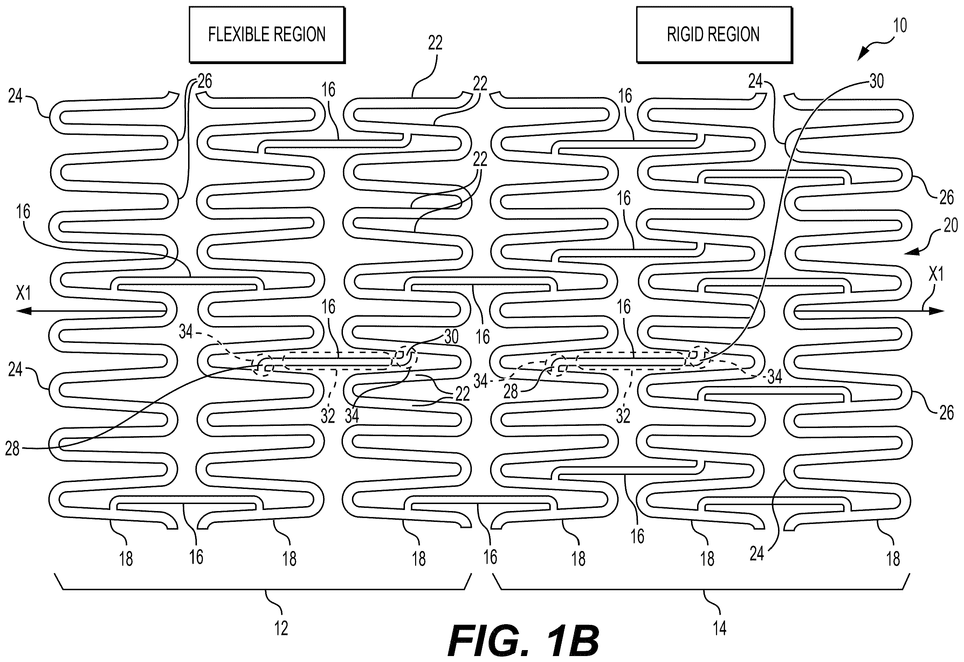

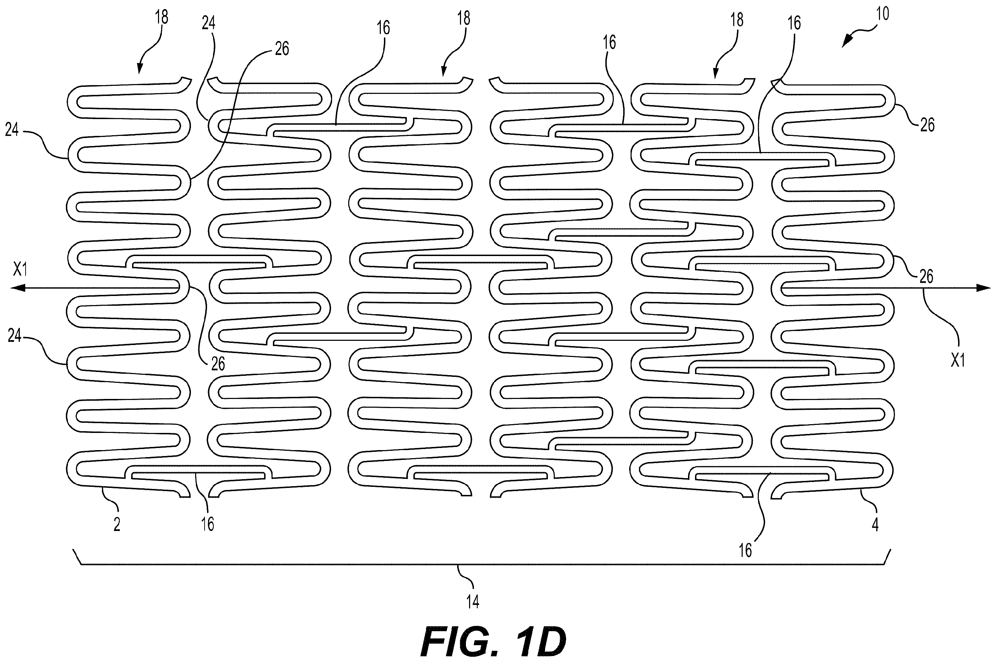

The stents 10, 110 of the present disclosure can be coated, covered, partially covered, fully encapsulated, partially encapsulated, or uncovered. In some examples, the members, tines, rings, and/or struts of a flexible portion 12, 112 and/or a rigid portion 14 (shown in FIGS. 1B and 1D) of the stents 10, 110 can be cut from a continuous tube by an automated cutting process, such as laser cutting. In some instances, portions of the stent 10, 110 can also be formed by connecting separate elongated members together and/or to other elements of the stent 10, 110 to form a tubular structure. For example, elongated members can be connected together or to other elements of the stents 10, 110 by ultrasonic welding, laser welding, or another suitable connecting process. Also, a plurality of tines or elongated members of the stents 10, 110 may be woven together to form portions of the stent 10, 110. Dimensions of the stents 10, 110, such as a longitudinal length L1 (shown in FIG. 1A) of the stents 10, 110, a length of the flexible portions 12, 112, a length of the rigid portions 14 of the stents 10, 110, a compressed diameter of the stents 10, 110, an expanded diameter D1 (shown in FIG. 1A) of the stents 10, 110, etc., can be selected based on various considerations including, but not limited to, the intended deployment location, intended delivery assembly and technique, and/or intended manner of use for the stents 10, 110.

As described in detail herein, the hinging mechanism of the present disclosure refers to portions of the stents 10, 110 having a reduced bending stiffness compared to other portions of the stents 10, 110 and/or compared to conventional stent designs, while maintaining radial stiffness and axial stiffness, such that the stents 10, 110 fulfill their intended purposes. The reduced bending stiffness may relate to a reduced amount of force required to bend, curve, or hinge the stents 10, 110 in a given direction. It is noted that the type of movement among bending, curving, and hinging will be understood by one skilled in the art such that these terms will be used interchangeably herein. The reduced bending stiffness may be relative to a variety of perspectives such as those just noted. For example, with regard to bending in a selected direction, the stents 10, 110 may have portions designed to have the reduced bending stiffness such that the stents 10, 110 can bend in the selected direction at these portions with a reduced amount of force as opposed to other portions that require a greater amount of force. In another perspective, the reduced bending stiffness may be relative to a circumference of the stents 10, 110. For example, within a selected circumference, the stents 10, 110 may have a reduced bending stiffness in one bending direction over another bending direction. As will be described in further detail below, the reduced bending stiffness may be achieved by creating a hinging axis that reduces the force required for the stents 10, 110 to hinge about the hinging axis over a different axis or other than about the hinging axis. The stents 10, 110 disclosed herein are configured to have sufficient radial stiffness to maintain patency of a body passageway or vessel, when the stents 10, 110 are deployed in the body lumen and exposed to normal body forces of the deployment location. The stents 10, 110 disclosed herein are also configured to have sufficient axial stiffness to substantially maintain a length of the stents 10, 110 during manufacturing (e.g., easier processing to manufacture the stent with an increased successful production rate), during delivery (e.g., increased column strength to accommodate for forces encountered during delivery to a target site), and/or during deployment (e.g., lower and more predictable foreshortening).

As used herein, "bending stiffness" refers to a structural member's resistance to deflection, such as deflection that occurs when a load is applied to a free end of the structural member. An example of "deflection" of a structural member is deformation of an end of a cantilever beam that occurs when a load is applied to the free end of the cantilever beam. A stent 10, 110 with high bending stiffness requires greater force to displace an end of the stent 10, 110 by a specified distance compared to a stent 10, 110 that is more flexible. Bending stiffness of an elongated member, such as a beam or stent, can be measured by a cantilever test, as is known in the art. The forces applied to the stents 10, 110 that cause the stents 10, 110 to bend may be dependent upon a variety of factors. For example, as those skilled in the stent manufacturing art will understand, the material of the stents 10, 110 or components thereof may contribute to the bending stiffness and subsequently the force required to bend the stents 10, 110. As will be described in further detail below, the configuration of the components of the stents 10, 110 may also contribute to the bending stiffness, particularly when the configuration allows for a relatively reduced amount of force to be required to bend the stents 10, 110.

By contrast, "radial stiffness" refers to resistance to deformation when a radially inwardly directed force is applied to a sidewall of the stents 10, 110. Stents 10, 110 with low radial stiffness may deform, compress, or collapse when radially inwardly directed forces, such as radially inwardly directed forces from surrounding tissues (e.g., a constricting or collapsing passageway or vessel) are applied to a sidewall of the stent 10, 110. The stents 10, 110 of the present disclosure are configured to have sufficient radial stiffness to avoid radial collapsing or radial compressing when the body passageway in which the stent 10, 110 is deployed constricts around the stent 10, 110 so that fluid flow through a lumen of the stent 10, 110 is preserved. The radial forces to which the stents 10, 110 may be exposed during normal use can be determined by those skilled in the stent manufacturing art and will vary based on the intended deployment location and/or use of the stents 10, 110. Accordingly, in accordance with the principles of the present disclosure, those skilled in the art may modify the geometric configuration, materials, and other characteristics of the stents 10, 110 disclosed herein to obtain sufficient radial stiffness so that the stent 10, 110 fulfills its intended purpose.

As also used herein, "axial stiffness" refers to resistance to compression or stretching along a given axis. In a particular manner, the axial stiffness along a main longitudinal axis of the stent 10, 110 (e.g., a central axis of a lumen of the stent 10, 110) may be referred to as a column strength, with particular regard to resisting compression along the main longitudinal axis. The stents 10, 110 of the present disclosure are configured to have axial stiffness or column strength sufficient to prevent an overall length of the stents 10, 110 from substantially changing during the various stages discussed above. The axial forces to which the stents 10, 110 may be exposed during normal use can be determined by those skilled in the stent manufacturing art and will vary based on the intended deployment location and/or use of the stents 10, 110. Accordingly, in accordance with the principles of the present disclosure, those skilled in the art may modify the geometric configuration, materials, and other characteristics of the stents 10, 110 disclosed herein to obtain sufficient axial stiffness or column strength so that the stent 10, 110 fulfills its intended purpose.

In some examples, the stents 10, 110 of the present disclosure comprise multiple hinging mechanisms or hinge points that allow the stents 10, 110 to flex or bend in different directions when subjected to a force reduced with respect to the force necessary to bend portions of the stents 10, 110 separated from the hinge points or where a hinge point is not present. As will be described in further detail below, the hinging mechanism or hinge point may correspond to creating a hinging axis through the stents 10, 110. Including hinging mechanisms or hinge points at different circumferential and/or axial locations provides a stent 10, 110 with a multi-directional hinging ability meaning that different portions of the stent 10, 110 bend easily in different directions selected, e.g., to permit the stents 10, 110 to conform to the anatomy of the target locations at which they are to be deployed and/or to reduce the overall bending stiffness of the stent 10, 110. The stent 10, 110 may therefore be configured to adopt non-linear shapes such as when deployed at a target location that requires the stent 10, 110 to accommodate a bent, curved, or hinged configuration. As used herein, a hinge point of the stent 10, 110 "bends more easily" in one direction than in another direction when a bending force required to bend the stent 10, 110 at the hinge point by a predetermined distance in one direction is less than a bending force required to bend the stent 10, 110 at the hinge point by the predetermined distance in another direction. That is, the stent 10, 110 may bend more easily in a hinged direction due to a relatively reduced amount of force. The multi-directional hinging mechanism allows the stent 10, 110 to bend or flex in multiple directions without kinking, thereby preventing a restriction of fluid flow through a lumen of the stent 10, 110. By avoiding or resisting kinking, the stent 10 can be deployed in various or multiple bent or curved configurations without restricting fluid flow through the stent 10, 110 nor reducing the ability of the stent 10 to maintain patency of the blood vessel.

In order to provide radial stiffness, axial stiffness, and kink resistance, the stents 10, 110 of the present disclosure include various geometric arrangements of expandable rings 18, 118, elongated connectors 16, 116a, 116b, and/or struts positioned to provide proper support for the rings 18, 118 to prevent the stent 10, 110 from collapsing (e.g., maintaining radial and axial integrity), while allowing easy bending in selected directions or multiple directions (e.g., maintaining flexibility). In particular, by selectively positioning the elongated connectors 16, 116a, 116b or struts between adjacent ones of the rings 18, 118, the stent 10, 110 can be configured to selectively modify the bending stiffness of respective portions of the stent 10, 110 to allow these portions to hinge or bend easily in at least one direction. Further, hinge points of the stent 10, 110 can be selectively positioned for different rings 18, 118 (e.g., through circumferential offsets on the rings 18, 118) creating the multi-directional hinging mechanism that reduces the bending stiffness of the stent 10, 110 in selected directions when compared to conventional stent designs, and allowing for the stent 10, 110 to hinge or bend in all directions at different locations along the stent 10, 110 because each direction may be achieved through a corresponding positioning of the elongated connectors 16, 116a, 116b around the rings 18, 118. Also, the stents 10, 110 disclosed herein are configured to provide predictable recoil properties after deployment to maintain proper wall apposition. In particular, the stents 10, 110 of the present disclosure that are placed in fenestrations should be strong enough to resist migration of the associated endo-graft and not collapse due to shear forces. It is noted that the elongated connectors 16, 116a, 116b may be included in the stents 10, 110 in a variety of manners and combinations to exhibit, for example, different shapes such as being straight, being angled, being curved, having a fusiform shape, having a funnel shape, etc. In a particular embodiment, depending on a location along a length of the stents 10, 110, the elongated connectors 16, 116a, 116b may have a corresponding shape (e.g., a fusiform shape for the elongated connectors 16, 116a, 116b at or near ends of the stents 10, 110 while a straight shape therebetween).

By contrast, conventional stainless steel stents, as known in the art, generally comprise multiple expandable rings arranged in series and connected together by multiple equidistantly spaced connectors, for example, four connectors positioned at 0 degrees, 90 degrees, 180 degrees, and 270 degrees around the rings of the conventional stent. The elongated connectors between different rings of the conventional stent can be axially aligned or nearly aligned creating, in effect, four lines of connectors extending the entire length or a portion of the length of the stent. The lines of connectors can be spaced apart by 90 degrees around the stent. The arrangement of connectors in the conventional stent provides a rather rigid stent that is well suited for supporting a vessel wall and preventing the stent from collapsing when deployed. However, the conventional stent with the equidistantly spaced elongated connectors does not bend easily and, if subject to enough force to cause the conventional stent to bend, may kink, such that a cross sectional area of the lumen decreases which restricts fluid flow through the lumen of the conventional stent.

According to the present disclosure, the stents 10, 110 comprising the multi-directional hinging mechanism disclosed herein are also configured to address certain difficulties encountered during delivery of conventional stents. As previously described, a balloon expandable stent is typically delivered using a balloon catheter to which the stent 10, 110 is crimped. During delivery, the stent 10, 110 should be configured to maintain adequate stent retention to the catheter so as not to become dislodged during placement. Further, balloon expandable stents 10, 110 should be configured to be delivered and positioned accurately without extensive foreshortening. The stents 10, 110 of the present disclosure, which are configured to flex or bend in multiple directions, can be easily and tightly crimped to a balloon expandable catheter reducing the likelihood that the stents 10, 110 will be become dislodged or slide off of the catheter during placement.

In some examples, the flexible portion of the stents 10, 110 disclosed herein includes groups or pairs of elongated connectors 116 positioned to exhibit a funnel shape. As described in further detail herein, the funnel shape is created by two adjacent ones of the elongated connectors 116 extending between a first ring and a second ring. Connection points at which adjacent connectors 116 are connected to the first ring are closer to one another circumferentially than are the connection points between these same connectors 116 and the second ring. Accordingly, each of the elongated connectors 116 or a segment thereof is angled or tapered (e.g., relative to a longitudinal axis of the stent 10, 110) such that the elongated connectors 116, when paired, form a funnel shape with the elongated connectors 16, 116 diverging from one another in the direction from the first ring to the immediately adjacent second ring. Each of the adjacent elongated connectors 116 of these pairs may, optionally, be angled relative to a central axis of the funnel configuration the longitudinal axis of the stent 10, 110 by an equal but oppositely directed amount (e.g., symmetric about a central axis of the funnel configuration which may, optionally, be parallel to a longitudinal axis of the stent 10, 110). As described in further detail herein, arranging the elongated connectors 116 in this funnel shape configuration provides for increased column strength, allows for easier processing, leads to lower and more predictable foreshortening, prevents kinking when the stent 110 bends, while also providing benefits of stent retention. In particular, this funnel configuration creates narrow hinge points on one ring element (i.e., the ring element where the connection points are close together) and a wide base on the adjacent ring element (i.e., the ring element with connection points that are farther apart). When moving from the narrow hinge point to the wide base, the angled segment of the elongated connectors 116 extend away from each other. The combination of the narrow hinge points with the wide base provides for both high flexibility at a hinging axis that is created adjacent the narrow hinge points and additional column strength compared to other bending stents with ring and connector configurations. Increased column strength is a benefit for manufacturing and end use of the stent 10, 110. As would be understood by those skilled in the art, to achieve the desired higher flexibility at the narrow hinge points, it is desirable, but not required, that the pairs of elongated connectors 16, 116 at the same location longitudinally along the stent 10, 110 have the narrower spacing between the connectors 16, 116 at the connection to the same one of the rings (e.g., the first ring) to which they are connected so that the funnels open wider for both pairs of elongated connectors 16, 116 at the same ring (e.g., the second ring). As would be understood by those skilled in the art, other arrangements of the directions of the funnel shapes of the connectors 16, 116 may be employed without the common directionality and these other arrangements will still increase the bending flexibility of the stent 10, 110.

### Multi-directional hinging stents

FIG. 1A shows an exemplary expandable stent 10 including a multi-directional hinging mechanism without the funnel configuration. The stent 10 is configured to radially expand from a radially retracted or radially compressed configuration to a radially expanded configuration, as illustrated in FIG. 1A. The stent 10 comprises a first or proximal end 2 and a second or distal end 4. The stent 10 can comprise one or more flexible segments or flexible portion(s) 12 (shown in FIGS. 1A-1C) that is configured to hinge at a predetermined position about a hinging axis. As used herein, the "flexible portion" can refer to portions of the stent 10 having a configuration selected so that the stent 10 is flexible and bends more easily than in the non-flexible portion(s) in at least one or multiple directions where the directions can be predetermined based on the configuration. By contrast, the previously described conventional stents are often axially rigid and resist bending in any direction and/or require substantially equal force to bend in any direction where that force is greater than the force the stent 10 may require to bend about the hinging axis. The flexible portion(s) 12 can be arranged so that different portions or segments of the stent 10 bend more easily in different directions, thereby producing the multi-directional hinging stent that bends easily in multiple directions. That is, the stent 10 may be configured with a stent design for the flexible portion(s) 12 to bend in predetermined directions, at predetermined locations along the stent 10, and in one or more directions due to the reduced amount of force that is required for the stent 10 to bend in the predetermined directions, at the predetermined locations than in other locations or directions. The multi-directional hinging allows the stent 10 to be deployed in a wide variety of positions and configurations. When deployed, the stent 10 is configured to maintain the integrity of its intended radial structure by providing a radial stiffness so that fluid flow through a lumen of the stent 10 is not obstructed when the stent 10 is exposed to body forces expected at the deployment location. As previously described, "radial stiffness" refers to resistance to radial deformation particularly to deformation of the stent 10 radially inward. A stent 10 with reasonable radial stiffness is able to resist collapsing and/or to maintain a shape and cross-sectional area even when external forces (e.g., forces created by a restricting or collapsing vessel or artery) constrict around the stent 10. In particular, the stents 10 disclosed herein should be sufficiently strong so that fluid flow through a lumen of the stent 10 is not substantially reduced or obstructed even when the body passageway or vessel in which the stent 10 is deployed begins to contract or collapse around the stent 10.

The stent 10 can also include one or more rigid sections or rigid portions 14 (shown in FIGS. 1B and 1D) connected to and axially aligned with the flexible portions 12. As used herein, a "rigid portion" of the stent 10 refers to a portion of the stent 10 having a configuration selected to resist bending in any direction and/or to portions or segments of the stent 10 where the force needed to bend the stent 10 is substantially equal in any direction. The rigid portion may substantially correspond to a rigid design of conventional stents with rings and connectors where the rigid design instead extends through the entire conventional stent. In contrast, the stent 10 may selectively incorporate one or more of the rigid portions 14 within the overall stent 10 along with one or more flexible portions 12 with the intent of preventing bending at these segments of the stent 10. A stent 10 comprising both a flexible portion 12 and a rigid portion 14 is shown in FIG. 1B. The rigid portion 14 comprises multiple axial or elongated connectors 16 or struts that are arranged to resist bending. For example, relative to a section of the flexible portions 12, the rigid portion 14 may comprise a greater number of the elongated connectors 16. By contrast, as will be described in further detail below, the flexible portion 12 can include the elongated connectors 16 arranged to resist bending in selected directions by generating a structure requiring a greater amount of force for the bending to occur (e.g., in an amount similar to the rigid portion 14), while requiring a reduced amount of force to permit bending in other directions.

The flexible portion(s) 12 and rigid portion(s) 14 of the stent 10 according to this embodiment may generally be formed from the same material. For example, the flexible portion(s) 12 and rigid portion(s) 14 can comprise members, tines, rings, and/or struts formed from suitable metal materials, such as stainless steel or cobalt chromium. The flexible portion 12 and/or rigid portion 14 can also comprise biocompatible polymers, absorbable polymers, and/or other biomaterials, as are known in the art. The portion(s) 12, 14 of the stent 10 may also be formed from a super-elastic material. An exemplary super-elastic material commonly used in the stent art is nickel titanium alloy (e.g., NITINOL). As is known in the art, NITINOL also has shape-memory properties that permit the imparting to NITINOL-containing structures of a memorized shape or configuration to which the structures will revert (after having been placed in a different shape or configuration when the shape memory properties of the NITINOL-containing structure are actuated (e.g., by being heated to a particular temperature (e.g., body temperature), etc.). However, shape-memory properties are not required for the present invention. Instead, NITINOL and similar materials may be used with the stents 10 disclosed herein to provide super-elasticity and enhanced flexibility, which may be beneficial for certain uses of the stents 10 disclosed herein. As noted above, the materials used for the elongated connectors 16 may define the amount of force required to bend the stent 10.

In many cases, the flexible portion(s) 12 and the rigid portion(s) 14 of the stent 10 are formed by laser cutting selected geometric patterns from a single tubular structure. In such cases, the flexible portion(s) 12 and rigid portion(s) 14 of the stent 10 are integrally formed and differ only in the orientation of the elongated connectors 16 and other geometric features of the stent 10. In other examples, the flexible portion(s) 12 and rigid portion(s) 14 can be formed as separate elements connected together using, for example, ultrasonic welding, adhesives, suturing, or other commonly used connecting techniques, as are known in the stent manufacturing art.

With continued reference to FIGS. 1A-1D, the stent 10 can comprise multiple radially expandable rings 18 aligned in series along a common axis X1, as shown in FIG. 1B, and defining a common lumen 20 of the stent 10, as shown in FIG. 1A, extending through the rings 18. Within the stent 10, the flexible portion(s) 12 and the rigid portion(s) 14 can comprise the rings 18. The rings 18 can be circular in shape defining a cylindrical shape for the lumen 20. In other examples, one or more of the rings 18 can be shaped as an oval, triangle, square, rectangle, polygon, or any other convenient regular or irregular shape, such that the lumen 20 has a corresponding cross-sectional shape along the axis X1. The rings 18 can be fully annular members that fully enclose the lumen 20 of the stent 10 or can include a gap or opening somewhere on the ring 18. Gaps or openings of the multiple rings 18 of the stent 10 can be aligned axially along a length of the stent 10 or can be offset from one another.

In some examples, the rings 18 comprise multiple linear segments 22 connected together end-to-end forming a substantially repeating pattern of peaks 24 and valleys 26 of the ring 18. As used herein, "substantially repeating" linear segments 22 can refer to units that are repeating but could accommodate minor interruptions in the repeating pattern as would be understood by those skilled in the art. Minor interruptions in the repeating pattern can be, for example, changes or substitutions to the repeating linear segments 22 of the ring(s) 18 that do not affect expansion of the ring(s) 18. For example, some linear segments 22 could be formed from curved portions (e.g., rather than connected straight segments). Also, some linear segments 22 could be longer or shorter than other linear segments 22 of the ring(s) 18. Thus, the arrangement of linear segments 22 of the rings 18 shown in FIGS. 1A-1D are not intended to be limited to a strict and exact repeating pattern of linear segments 22. For example, a ring 18 that includes repeating linear segments 22, but with one or several minor interruptions to the repeating pattern, is considered to be within the scope of the present disclosure.

Each of the rings 18 in FIGS. 1A-1D includes sixteen linear segments 22 defining eight peaks 24 and eight valleys 26. However, the number of linear segments 22, peaks 24, and valleys 26 is not intended to be limiting, and the rings 18 can have any number of linear segments 22, e.g., fewer than sixteen or more than sixteen linear segments 22, within the scope of the present disclosure. Further, in some examples, different rings 18 of the stent 10 may include different numbers of linear segments 22. For example, rings 18 near a middle of the stent 10 may include sixteen linear segments 22, while rings 18 near ends 2, 4 of the stent 10 may include more linear segments (e.g., twenty or more linear segments 22 per ring 18). While rings 18 can be provided in a wide variety of configurations and orientations, for simplicity, as used herein, peaks 24 of the ring 18 refer to portions of the ring 18 closer to the proximal end 2 of the stent 10. Valleys 26 refer to portions of the ring 18 that are closer to the distal end 4 of the stent 10. When the stent 10 is in the radially compressed configuration, the rings 18 are radially compressed, meaning that a diameter of the ring 18 is relatively smaller, and an amplitude of the ring 18 (e.g., an axial distance between a peak 24 and a valley 26 of the ring 18) is relatively larger. As the stent 10 transitions from the radially compressed configuration to the radially expanded configuration (shown in FIG. 1A), the diameter D1 of the ring 18 increases while the amplitude between the peaks 24 and valleys 26 of the ring 18 decreases. For example, the diameter D1 of the rings 18 of the stent 10 may increase by 2.0 mm, 4.0 mm, 8.0 mm or more, as the stent 10 is expanded.

The rings 18 of the stent 10 can be arranged in a variety of configurations, as are known in the art. In some examples, as shown in FIGS. 1A-D, the rings 18 are arranged in a "peak-to-valley" configuration, where peaks 24 of a first one of the rings 18 is axially aligned or substantially aligned with valleys 26 of a second one of the rings 18 immediately adjacent to the first ring 18. As used herein, a first ring is "immediately adjacent" to second ring when there are no other rings between the first ring and the second ring. It is understood that there may be other structural elements (e.g., elongated connectors, struts, portions of a cover) between the first ring and the second ring. As used herein, "axially aligned" refers to points on different rings 18 of the stent 10 that are co-linear (e.g., on a same line) along a line that is parallel to (e.g., does not overlap or intersect with) the common axis X1 of the stent 10.

In other examples, the rings 18 can be arranged "peak-to-peak", where a peak 24 of a particular ring 18 is axially aligned with a peak 24 of an immediately adjacent ring 18. Thus, in comparison to the "peak-to-valley" configuration, the "peak-to-peak" configuration may arrange the rings 18 so that a line may pass through the peaks 24 in consecutive ones of the rings 18 that is parallel to the axis X1. A line passing through the valleys 26 in consecutive ones of the rings 18 is also parallel to the axis X1. In other examples, peaks 24 and valleys 26 of a particular ring 18 can be circumferentially offset from (e.g., not aligned with) both peaks 24 and valleys 26 of an immediately adjacent ring 18. In some examples, all rings 18 of the stent 10 can be arranged in the same orientation (e.g., all the rings 18 are peak-to-peak or all the rings 18 are peak-to-valley). In other examples, a stent 10 may include some portions or segments with the rings 18 arranged peak-to-peak, other portions or segments with the rings 18 arranged peak-to-valley, still other portions or segments with the rings 18 arranged offset with no circumferential alignment, or any combination thereof.

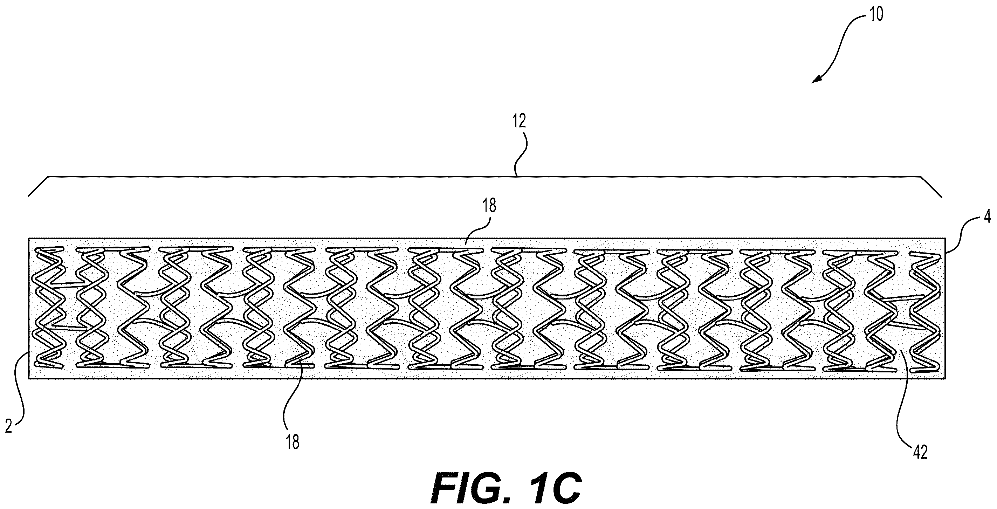

In some examples, the stent 10 is a covered stent. For example, all or any number of portions of the stent 10 can be covered by a cover 42 outside and/or inside the stent 10. An exemplary covered stent 10, including features of the present disclosure, is shown in an expanded configuration in FIG. 1C. As shown in FIG. 1C, the cover 42 encloses the entire stent 10 including both the flexible portion 12 and the rigid portion 14 of the stent 10. In other examples, only some sections or portions of the stent 10 may be covered while other sections or portions of the stent 10 are bare. For example, in one embodiment only the rigid portion 14 of the stent 10 will be covered by the cover 42, while the flexible portion 12 is bare, or vice versa. In other examples, the cover 42 may partially enclose both the flexible portion 12 and the rigid portion 14 of the stent 10, while segments or portions near the ends 2, 4 of the stent 10 are bare. The cover 42 can be formed from, for example, a sheet or film of a biocompatible material. The sheet or film can be configured to protect vessel walls defining the body passageway or vessel from edges of the rings 18 and other elements of the stent 10. In some examples, the cover 42 can be formed from a low friction material configured to protect the stent 10 and to reduce or prevent biological materials from adhering to portions of the stent 10. For example, the cover 42 can be formed from a low friction and/or hydroscopic material, such as expandable Polytetrafluoroethylene (ePTFE). In some examples, the material of the cover 42 is elastic and capable of stretching without breaking as the stent 10 expands. However, the elasticity of the material of the cover 42 should not be so strong as to cause the stent 10 to collapse from the expanded configuration back to the compressed configuration. In some examples, the cover 42 can be designed to also perform a secondary function, such as providing hemostasis. Also, the cover 42, the rings 18, and/or the elongated connectors 16 of the stent 10 can be coated, covered, and/or impregnated with a therapeutic agent, such as heparin.

The decision of whether the rings 18 are peak-to-peak, peak-to-valley, or offset is often based on whether the stent 10 is covered or bare, and, if covered, based on material characteristics of the cover 42. In particular, the stents 10 with the rings 18 arranged peak-to-valley can cause the cover 42 to stretch as the rings 18 expand and rotate or twist, because the peaks 24 of one ring 18 tend to rotate away from the valleys 26 of the immediately adjacent ring(s) 18 as the stent 10 expands. If the stent 10 is bare or if the cover material is flexible or elastic, then this movement of the peaks 24 and the valleys 26 may be acceptable. However, for the stents 10 comprising the cover 42 formed from a stiff material that does not stretch easily, rotation of the peaks 24 away from the valleys 26 could cause the cover 42 to tear. In such cases, using rings 18 arranged in a peak-to-peak configuration or an offset configuration may extend the usable life of the stent 10.

The flexible portions 12 and the rigid portions 14 of the stent 10 further comprise the elongated connectors 16 extending between the rings 18. As shown in FIG. 1B, each of the elongated connectors 16 can comprise a proximal or first end 28 connected to one of the rings 18 at a connection point and a second end 30 connected to an immediately adjacent ring 18 at another connection point. The elongated connectors 16 can include one or more straight segments, such as a straight segment 32. In some examples, the elongated connectors 16 can also include bent portions, curved portions, pigtailed portions, or combinations thereof, configured to bend or unfold as the stent 10 transitions between the compressed configuration and the expanded configuration. For example, as shown in FIG. 1B, elongated connectors 16 can include bent portions 34 near the ends 28, 30 of the elongated connector 16. The straight segment 32 is disposed between the bent portions 34 of the elongated connector 16. As illustrated in FIGS. 1B and 1D of the stent 10, there can be different shapes of the elongated connectors 16. In a first type of the elongated connectors 16, the bent portions 34 may extend from the ends of the straight segment 32 in the same direction, forming a substantial C-shape or reverse C-shape. In a second type of the elongated connectors 16, the bent portions 34 may extend from the ends of the straight segment 32 in opposite directions, forming a substantial S-shape or reverse S-shape. Also, while not shown in FIG. 1B, some or all of the elongated connectors 16 can be branched, connecting to a particular ring 18 at two or more distinct connection points. Elongated connectors 16 of the flexible portion(s) 12 or rigid portion(s) 14 of the stent 10 can be generally identical or can have different characteristics such as differing widths to impart different structural characteristics for the stent 10. For example, thinner or narrower elongated connectors 16 may be used in regions of the stent 10 intended to be more flexible. Thicker or wider elongated connectors 16 can be used in regions of the stent 10 intended to be more rigid. In some examples, the ends 28, 30 of the elongated connectors 16 are connected to linear segments 22 of the rings 18 near a middle point of the linear segments 22 (e.g., approximately an equal distance between the peak 24 and the valley 26 of the ring 18). In other examples, elongated connectors 16 can be connected to the rings 18 closer to the peaks 24 or valleys 26 of the ring 18.

The number and position of the elongated connectors 16 generally influences the bending stiffness of the stent 10 or stent segment and, in particular, influences in which direction(s) the stent 10 or stent segment can bend with a reduced amount of force (i.e., in which direction the stent 10 (at this location) shows reduced bending stiffness). For example, as shown in the flexible portion 12 in FIG. 1B, in order to provide a suitable bending stiffness for the stent 10 to bend in a predetermined direction with the reduced amount of force, the elongated connectors 16 extending between a particular ring 18 and an immediately adjacent ring 18 are spaced apart by 180 degrees. Further, the substantially straight segment 32, for each elongated connector 16 extending from a particular ring 18 to an immediately adjacent ring 18, is axially and/or circumferentially offset from any other substantially straight segment 32 of a further one of the elongated connectors 16 extending from the particular ring 18. The rigid portions 14 of the stent 10 comprise the elongated connectors 16 arranged like the elongated connectors 16 of the previously described conventional stents. For example, the rigid portions 14 of the stent 10 can include multiple elongated connectors 16 extending between a particular ring 18 and an immediately adjacent ring 18 where a number of the elongated connectors 16 in the rigid portions 14 is greater than a number of the elongated connectors 16 in the flexible portions 12. The multiple elongated connectors 16 can be equidistantly spaced around the stent 10. For example, the elongated connectors 16 in the rigid portion 14 are spaced apart by 90 degrees from any immediately adjacent one of the elongated connectors 16. The elongated connectors 16 of the rigid portion(s) of the stent 10 can be axially and/or circumferentially offset, as shown in FIGS. 1B and 1D, or axially aligned to impart additional rigidity.

As previously described, FIG. 1D shows a rigid portion 14 of a stent 10. As in previous examples, the rigid portion 14 comprises multiple expandable rings 18 aligned in series along the common axis X1. Further, the rigid portion 14 includes the elongated connectors 16 extending between a particular ring 18 and an immediately adjacent ring 18. As shown in FIG. 1D, the stent 10 can include four elongated connectors 16 between each ring 18 and the immediately adjacent ring 18 that are equidistantly spaced about the circumference of the rings 18, such that each of the elongated connectors 16 is separated from adjacent ones of the elongated connectors 16 of a particular ring 18 by about 90 degrees. As previously described, the four elongated connectors 16 for each ring 18 are positioned to prevent the rigid portion 14 of the stent 10 from bending or hinging in any direction by requiring a greater amount of force to bend the stent 10 in the rigid portion 14 relative to an amount of force required to bend the stent 10 in the flexible portion 12, specifically about a hinging axis in the flexible portion 12.

### Multi-directional hinging stents with funnel shaped connectors

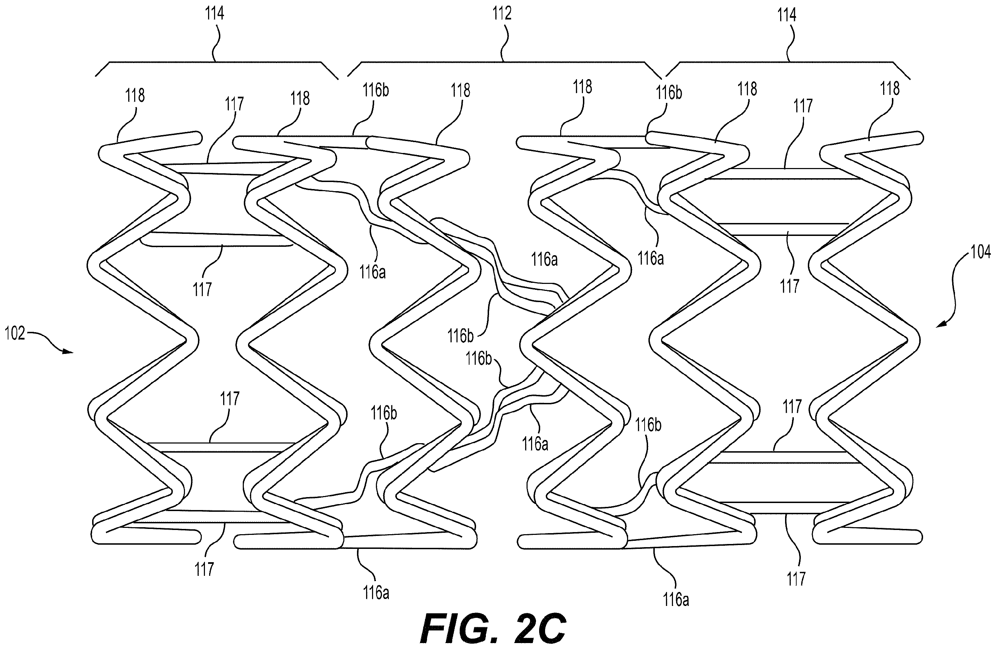

As previously described, stents 110 of the present disclosure can include elongated connectors 116a, 116b arranged in funnel configurations (e.g., exhibit a funnel-like shape) to form a respective hinging axis about which the stents 110 may bend with a relatively reduced amount of force. The elongated connectors 116a, 116b exhibiting the funnel shape are configured to have excellent flexibility, meaning that the stents 110 can deform and bend without kinking, while maintaining significant column strength compared to other multi-directional hinging stents. Also, as described in further detail herein, the elongated connectors 116a, 116b exhibiting the funnel shape can be positioned at a variety of circumferential positions about the stent 110 in order to tailor flexibility of the stent 110 for particular applications. Further, the stents 110 can include one or more rigid portions 114 (further described with regard to FIG. 2C) connected to flexible portions 112 to provide a stent 110 that bends or flexes in some regions and is rigid in other regions . The rigid portions 114 of the stent 110 may be substantially similar to the rigid portion 14 of the stent 10 in providing an increased rigidity. For example, the elongated connectors 16 described above with regard to FIGS. 1B and 1D and as configured in the rigid portion 14 may be included in the stent 110. Specifically, as described below in FIG. 2C, the stent 110 may include elongated connectors 117 in the rigid portion 114.

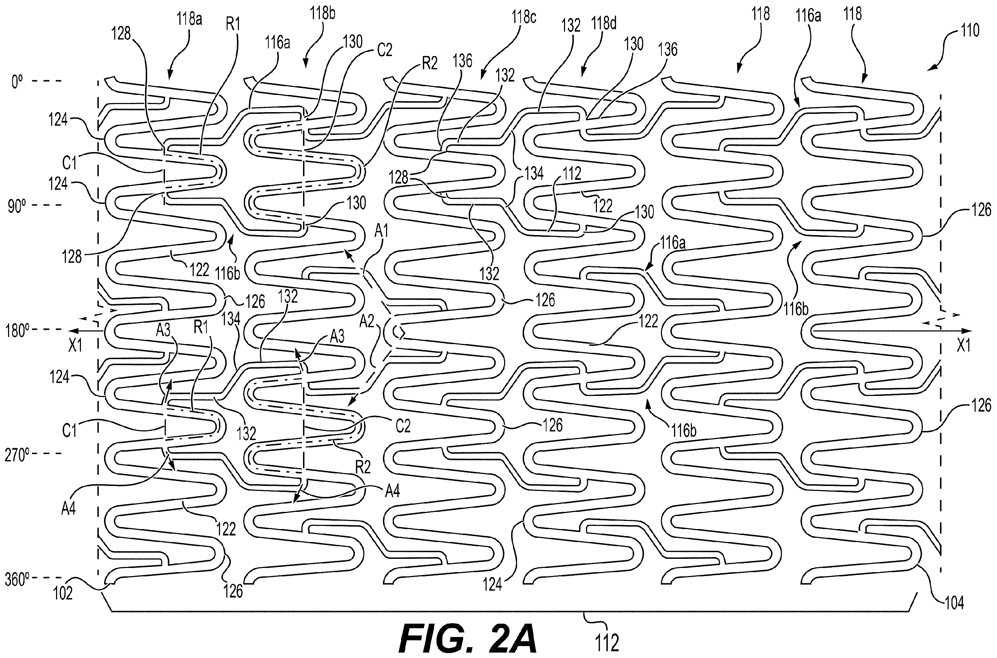

Detailed views of exemplary flexible portions 112 of expandable stents 110 including connectors 116a, 116b that exhibit the funnel shape are shown in FIGS. 2A-4C. As previously described, the connectors 116a, 116b that form or create the hinging axes and exhibit a funnel shape include a narrow hinge point on one of the rings, such as a first ring 118a, of the stent 110 and a wide base on an immediately adjacent ring, such as a second ring 118b, of the stent 110. The hinging axis created by the connectors 116a, 116b extends through a point adjacent and proximate the narrow hinge point, as described and illustrated below. The narrow hinge point provides flexibility, allowing the stent 110 to bend easily and without kinking. The wide base between the connectors 116a, 116b and the immediately adjacent or second ring 118b provides additional column strength that resists longitudinal deformation of the stent 110. As would be understood, the connectors 116a, 116b at any given location along the axis X1 are, in certain embodiments, oriented so that the narrow hinge points for each pair of adjacent connectors 116a, 116b are the same around the circumference of the stent 110. This ensures that, for these embodiments, the bendability of the stent 110 in the selected direction at this longitudinal location will be enhanced by alignment of the narrow hinge points of the pairs of connectors 116a, 116b around the circumference. As would be understood by those skilled in the art, this uniformity in the alignment of the narrow hinge points for the pairs of adjacent connectors 116a, 116b around the circumference of the stent 110 is optional and, if this uniformity is not maintained around the circumference, the resulting stent 110 will still have enhanced bendability.

As in previous examples, the stent 110 is an expandable stent 110 configured to radially expand from a compressed configuration to an expanded configuration. As shown in FIG. 2A, the stent 110 comprises multiple radially expandable rings 118 aligned in series along a common axis X1 and defining a common lumen of the stent 110. The flexible portion 112 can include a repeating pattern of radially expandable rings 118 and elongated connectors 116a, 116b extending between the rings 118, similar to previous examples. For convenience, in FIGS. 2A-4C, the rings 118 are identified as a proximal-most or first ring 118a, a second ring 118b, a third ring 118c, and a fourth ring 118d. As shown in FIGS. 2A-4C, other rings of the flexible portion 112 are identified generally as rings 118. However, the number of rings 118 is not intended to be limiting. Instead, the stent 110 may include any number of rings. In some examples, the stent 110 can include a repeating pattern of fewer than or more than four rings 118. For example, a stent 110 may include a repeating pattern of two or three rings 118 and elongated connectors 116a, 116b extending from the rings 118. In other examples, each ring 118 of the flexible portion 112 may be different from any other ring 118 of the flexible portion 112. For example, the connectors 116a, 116b can extend from a different position on each ring 118 of the flexible portion 112 of the stent 110 creating a stent 110 with unique multi-directional flexibility. In other examples, all rings 118 of the stent 110 may be identical with elongated connectors 116a, 116b extending from a same position on each ring 118 of the flexible portion 112.

Each ring 118 can include multiple linear segments 122 connected together end-to-end forming peaks 124 and valleys 126 of the rings 118. While the stents 110 can be provided in any configuration or orientation, in the present disclosure, peaks 124 refer to portions of the rings 118 closer to a proximal end 102 of the stent 110 and valleys 126 refer to portions of the rings 118 closer to the distal end 104 of the stent 110.

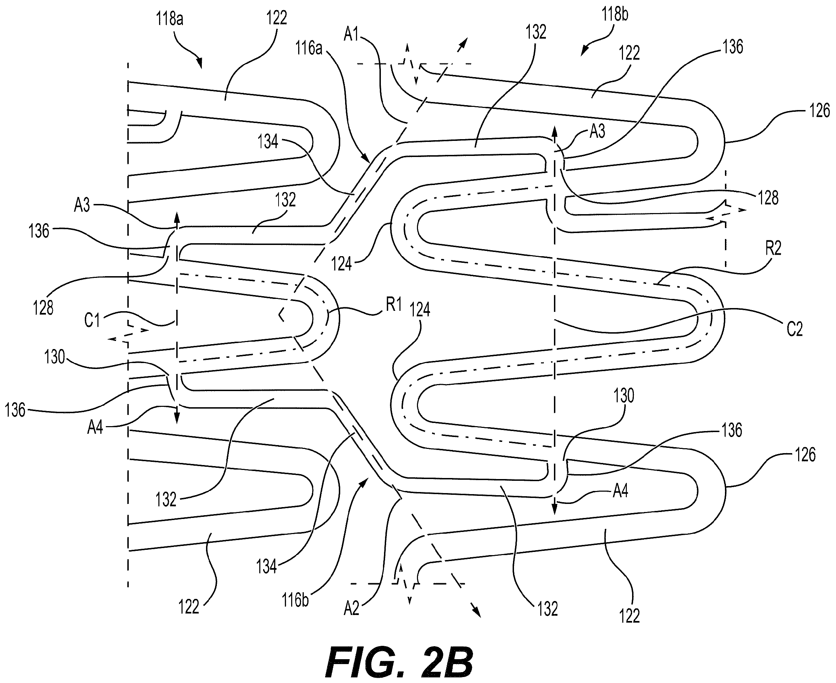

As shown in FIGS. 2A and 2B, the rings 118 are provided in a peak-to-peak orientation with the peaks 124 of the particular ring, such as the first ring 118a, axially aligned with the peaks 124 of the immediately adjacent or second ring 118b. The rings 118 can include multiple linear segments 122, peaks 124, and valleys 126 as well as any number of the linear segments 122, peaks 124, and valleys 126. For example, the rings 118 can include at least six peaks 124 and six valleys 126. In other examples, the rings 118 can include at least eight peaks 124, eight valleys 126, and sixteen linear segments 122 between the peaks 124 and the valleys 126. In other examples, the rings 118 can include an odd number of peaks 124 and/or valleys 126. Further, in some examples, different rings 118 of the stent 110 can include different numbers of peaks 124, valleys 126, and/or linear segments 122 than other rings 118 of the stent 110. The rings 118 may also have the various properties described above with regard to the rings 18 as well as the manner in which the rings 118 are axially and/or circumferentially aligned (e.g., peak-to-valley, offset, etc.).

The stent 110 also includes the multiple elongated connectors 116a, 116b extending between the rings 118 that form the funnel shape. As in previous examples, the rings 118 and/or elongated connectors 116a, 116b of the stent 110 can be formed from a rigid material, such as stainless steel, cobalt chromium, a nickel-titanium alloy, or a biocompatible polymer, the material at least partially contributing to the amount of force required to bend the stent 110. The stent 110 can be covered (e.g., covered with a cover formed from polytetrafluoroethylene (PTFE) or expanded polytetrafluoroethylene (ePTFE)), partially covered, or uncovered, in a manner substantially similar to that described above with regard to the stent 10. Also, portions of the stent 110 can be coated with a drug or another therapeutic agent as would be understood by those skilled in the art.

The elongated connectors 116a, 116b can be positioned about a circumference of the rings 118 to allow the stent 110 to bend or flex in one or more desired directions. As used herein, a "position" on a ring 118 refers to an arcuate distance around the circumference of the ring 118 measured from an initial or 0 degree position around the ring to a final position of just less than 360 degrees. For convenience, the "position" or arcuate distance is described herein as being measured in degrees. The "position" or arcuate distance could also be measured or described in terms of a position on a face of a clock (e.g., 12 o'clock, 3 o'clock, 6 o'clock, etc.) or in terms of a number of linear segments 122 of the ring 118 separating the elongated connectors 116a, 116b. As illustrated in FIG. 2A, the positions are shown in the flattened view. The circumference of the rings 118 now being shown as a linear measure, the positions are defined along this line for every 90 degrees where the 0 position and the 360 position would be the same when the stent 110 is in its circular cross-sectional orientation.

The stent 110 can also include one or more rigid portions 114 that do not bend or flex easily in a desired direction and/or which do not bend or flex easily in any direction. For example, a rigid portion 114 of the stent 110 can include equally spaced axial struts positioned at 0 degrees (12 o'clock), 90 degrees (3 o'clock), 180 degrees (6 o'clock), and 270 degrees (9 o'clock) around the rings 118 to stiffen the stent 110 and limit its bending or flexing in any direction therein. In a further example, as illustrated in FIG. 2C, the stent 110 may include a plurality of different types of elongated connectors based on whether the section of the stent 110 is the rigid portion 114 or the flexible portion 112. As shown, the stent 110 may include a first rigid portion 114 toward the proximal end 102 and a second rigid portion 114 toward the distal end 104 with a flexible portion 112 therebetween. This particular configuration including the rigid portions 114 at the ends of the stent 110 with the flexible portion 112 in the middle of the stent 110 is only for illustrative purposes. The number of rigid portions 114 and flexible portions 112 and the pattern in which these flexible and rigid portions are arranged longitudinally are also only for illustrative purposes. As described above, the flexible portion 112 may include the elongated connectors 116a, 116b (e.g., paired to exhibit the funnel shape). The rigid portion 114 in the stent 110 of FIG. 2C may include elongated connectors 117. The elongated connectors 117 may be substantially similar to the elongated connectors 16 described above in regard to FIGS. 1A-D. More specifically, as with the rigid portion 14 of the stent 10, the elongated connectors 117 may exhibit the same shape as the elongated connectors 16 and also be equally spaced apart (e.g., every 90 degrees).

The flexible portion 112 of the stent 110 includes a hinging capability along a predetermined hinging axis formed from one or multiple pairs or groups of two elongated connectors between two particular ones of the rings 118 of the stent 110, such as the first elongated connector 116a and the second elongated connector 116b. In an exemplary embodiment, two pairs of the elongated connectors (e.g., a first pair including a first one of the first elongated connector 116a and a first one of the second elongated connector 116b and a second pair including a second one of the first elongated connector 116a and a second one of the second elongated connector 116b) may be positioned on opposite sides of one another on adjacent rings 118s when the stent 110 is in the expanded configuration (e.g., 180 degrees apart around a circumference of the stent 110 such as at 0 degrees and 180 degrees or at 45 degrees and 225 degrees). The two pairs of the first connectors 116a and the second connectors 116b are oriented such that both of the narrow hinge points of the funnel shape of each pair are on a first one of the rings 118 while both of the wide bases of the funnel shape of each pair are on a second one of the rings 118. The two pairs of the first connectors 116a and the second connectors 116b can be positioned to create a hinging axis extending through the pairs adjacent and proximate the narrow hinge points such that a bending force required to bend the stent 110 by a predetermined distance in one direction about the hinging axis is less than a bending force required to bend the stent 110 by the predetermined distance in another direction. The stent 110 may also provide multi-directional hinging by positioning further two pairs of the elongated connectors at a different pair of the rings 118 and at a different circumferential position on the stent 110 (e.g., 90 degrees offset relative to the other pair of elongated connectors). This further two pairs of the elongated connectors may create a further hinging axis extending through the further pairs adjacent the narrow hinge points of these two pairs. Therefore, the stent 110 may be configured to hinge multi-directionally such as along a first hinging axis via the first two pairs of the elongated connectors and along a second hinging axis via the second two pairs of the elongated connectors at a different longitudinal location. Those skilled in the art will understand that any number of pairs of the elongated connectors may be positioned along the stent 110 to generate any number of hinging axes so that the stent 110 may bend in any number of directions along its length. For example, another two pairs of elongated connectors may be positioned at a different pair of rings 118 at another circumferential position of the stent 110 (e.g., 45 degrees offset, 30 degrees offset, 15 degrees offset, etc.).

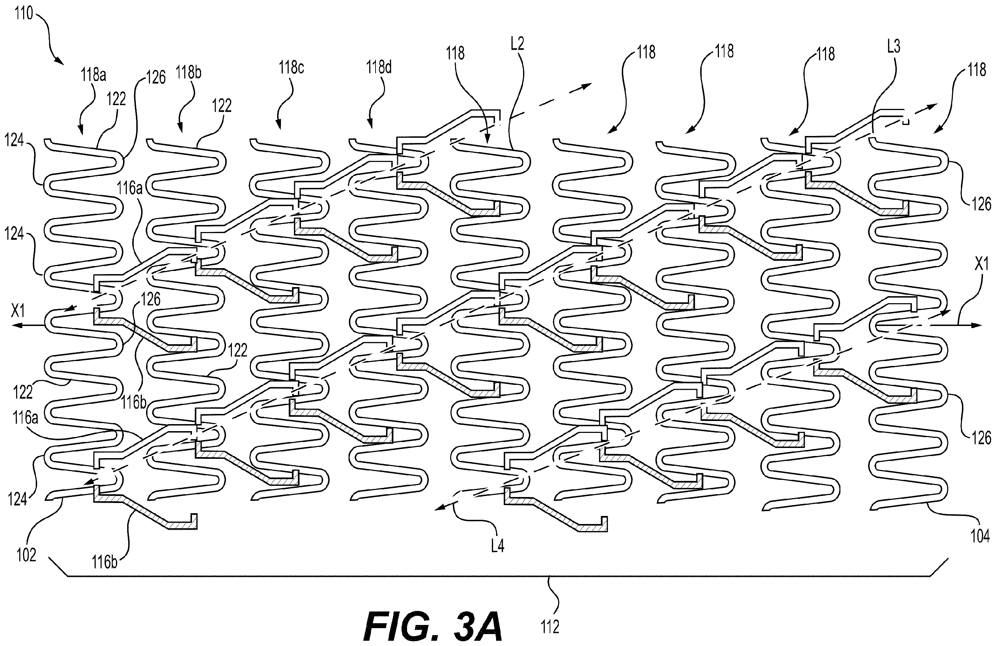

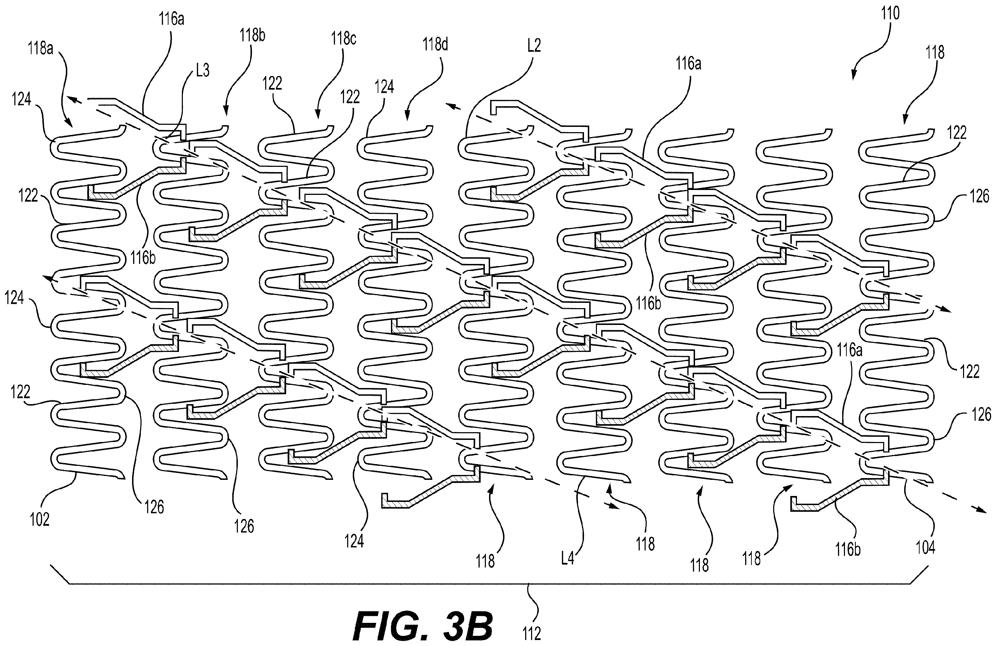

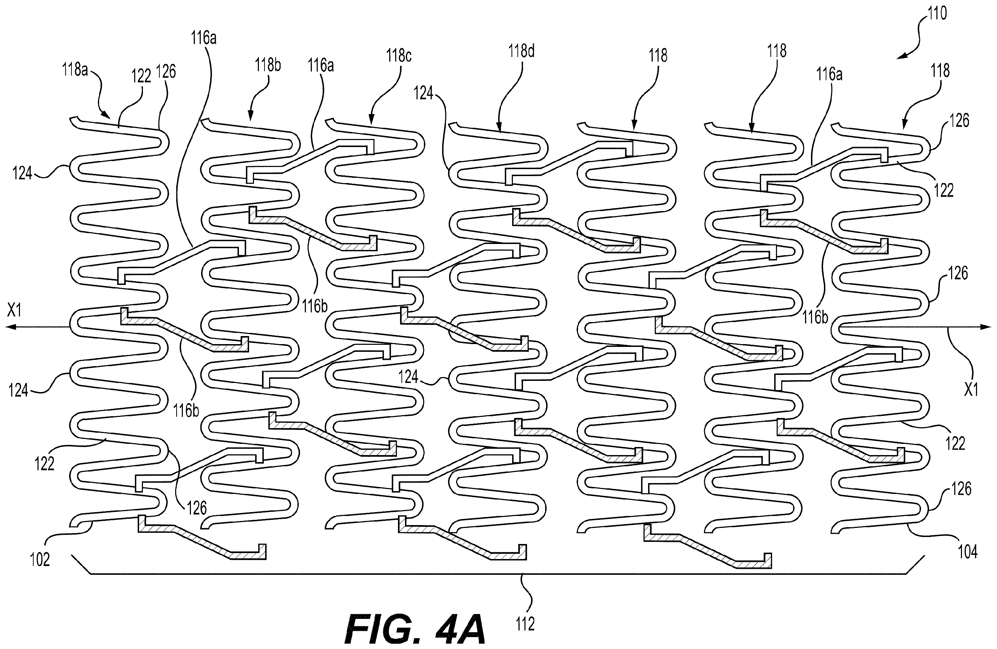

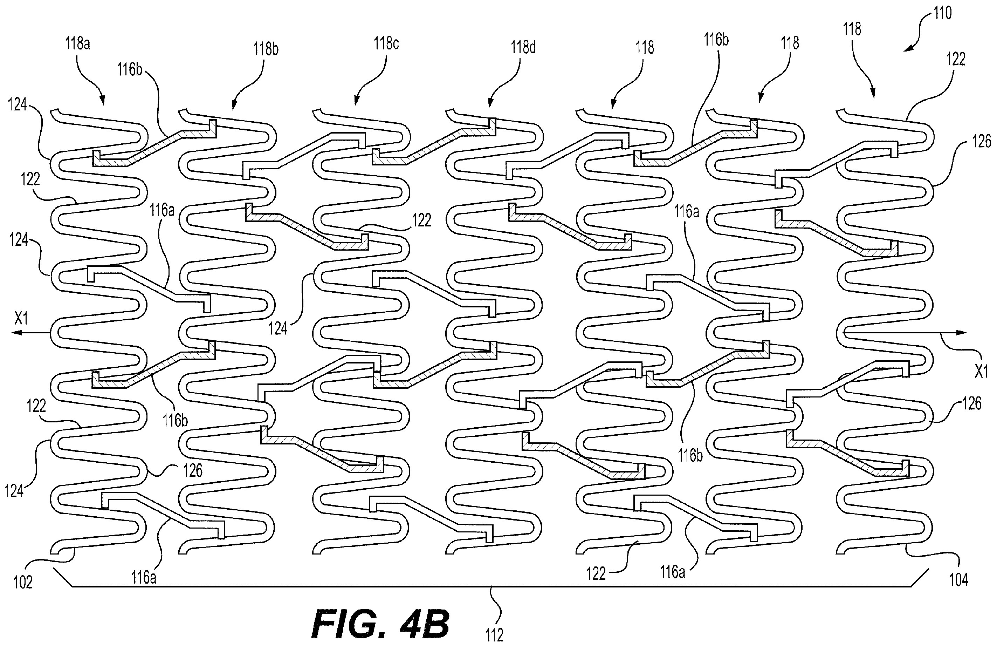

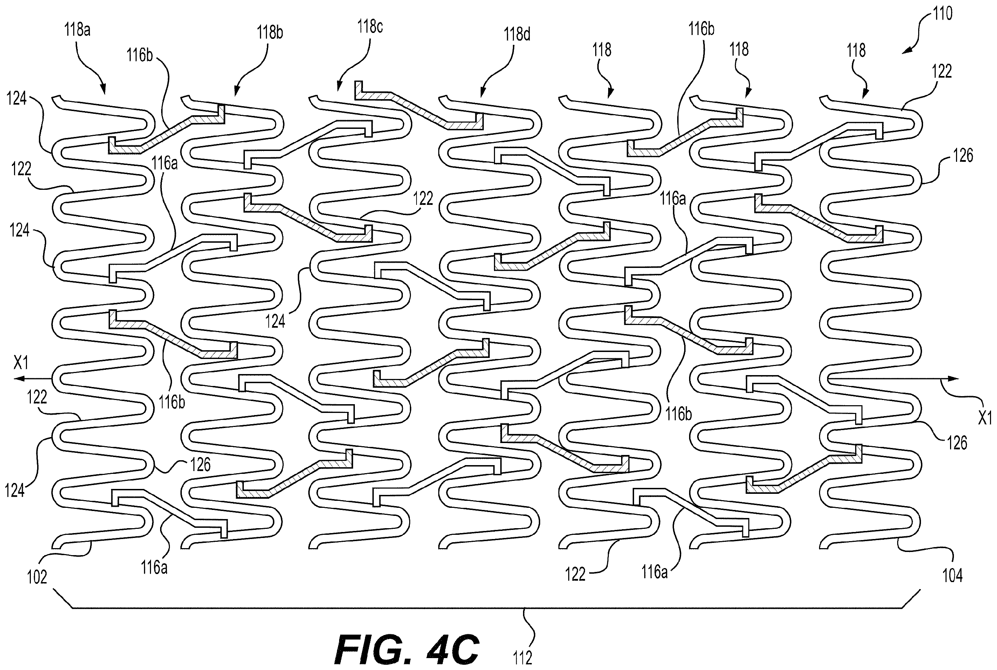

For the flexible portions 112 including the elongated connectors 116a, 116b exhibiting the funnel shape that creates a hinging axis, in some examples, all of the connectors 116a, 116b of the flexible portion 112 can be oriented in a same direction or orientation, as shown in FIGS. 3A, 3B, and 4A. In other examples, the flexible portion 112 can include the elongated connectors 116a, 116b exhibiting the funnel shape in different orientations, as shown in FIGS. 2A, 4B, and 4C. For example, one or more circumferential groups of the connectors 116a, 116b can be oriented with the narrow hinge points of the funnel shape being positioned closer to the proximal end 102 of the stent 110 while other circumferential groups of the connectors 116a, 116b can be oriented with the wide base of the funnel shape being positioned closer to the proximal end 102 of the stent 110. Also, the elongated connectors 116a, 116b can be uniformly distributed or unevenly spaced throughout the flexible portion 112 of the stent 110, circumferentially, axially, or a both, depending on the desired application.

Examples of the connectors 116a, 116b that have the funnel shape are shown in FIGS. 2A and 2B. As shown in FIGS. 2A and 2B, the funnel shape of the elongated connectors 116a, 116b is tapered having a narrow side (e.g., the narrow hinge point described above) and a wide side (e.g., the wide base described above). A width of the narrow side of the funnel shape is shown by a shortest distance (shown by line C1 in FIGS. 2A and 2B) about a circumference of the stent 110 between a connection point 128 between the first elongated connector 116a and the first ring 118a and a connection point 130 between the second elongated connector 116b and the first ring 118a. The width of the wide base of the funneled hinge is shown by a shortest distance (shown by line C2 in FIGS. 2A and 2B) about a circumference of the stent 110 between a connection point 128 between the first elongated connector 116a and the second ring 118b and a connection point 130 between the second elongated connector 116b and the second ring 118b. The distance C1 of the narrow side of the funnel shape is less than the distance C2 of the wide side of the funnel shape. As used herein, the "distance about a circumference" or "circumferential distance" refers to a length of an arc extending over an outer surface of the stent 110 in a plane that is orthogonal to the common axis X1 of the stent 110.

In a similar manner, as shown in FIGS. 2A and 2B, a shortest distance (shown by line R1 as a ring length in FIGS. 2A and 2B) along the first ring 118a between the connection points 128, 130 between the first connector elongated 116a and the second elongated connector 116b and the first ring 118a is less than a shortest distance (shown by line R2 as a ring length in FIGS. 2A and 2B) along the immediately adjacent ring or second ring 118b between connection points 128, 130 between the first elongated connector 116a and the second elongated connector 116b and the second ring 118b. As used herein, the ring length being a distance "along" a ring 118a, 118b refers to a length of wire or tube forming a portion of the ring 118a, 118b between the connection points 128, 130. For example, as shown in FIGS. 2A and 2B, the distance R1 along the ring 118a between connection points 128, 130 of the first ring 118a includes portions of two linear segments 122 separated by a valley 126 of the ring 118a. By contrast, the distance R2 between the connection points 128, 130 of the second ring 118b along the adjacent or second ring 118b includes portions of multiple linear segments 122, as well as two peaks 124 and a valley 126. Accordingly, the distance R2 is substantially longer than the distance R1.

In some examples, the connection points 128, 130 between the elongated connectors 116a, 116b and the rings 118a, 118b are at a substantially middle point of linear segments 122. For example, the connection points 128, 130 between the first ring 118a and the first and second connectors 116a, 116b can be positioned at a middle point of adjacent linear segments 122 separated by a valley 126. In a similar manner, the connection points 128, 130 between the first elongated connector 116a and the second elongated connector 116b and the immediately adjacent or second ring 118b can be positioned at a substantially middle point of non-adjacent linear segments 122 of the second ring 118b. However, the connection points 128, 130 need not be positioned at middle points of linear segments 122. Instead, in other examples, the connection points 128, 130 between the first elongated connector 116a and the second elongated connector 116b and the rings 118a, 118b can be at points on linear segments 122 that are closer to either the peak 124 or the valley 126 of the ring 118a, 118b. In other examples, the connectors 116a, 116b can be connected directly to a peak 124 or valley 126 of one or both of the rings 118a, 118b. For illustrative purposes, particularly in utilizing rounded figures for ring lengths corresponding to a length of the linear segments 122, the description below relates to when the connection points 128, 130 are positioned at the middle point of the linear segments 122.

As noted repeatedly above, the elongated connectors 116a, 116b are positioned and oriented to exhibit the funnel shape. In particular, the elongated connectors 116a, 116b can each comprise two axially extending segments 132 and an angled segment 134 between the two axially extending segments 132. The axially extending segments 132 of the elongated connectors 116a, 116b generally extend in a direction parallel or substantially parallel (e.g., within about 5 degrees of parallel) to the common axis X1 of the stent 110. However, this angular orientation is only for illustrative purposes and the axially extending segments 132 may extend in a non-parallel angular orientation. By contrast, the angled segments 134 of the connectors 116a, 116b are angled or skew relative to the common axis X1 of the stent 110. For example, as shown in FIGS. 2A and 2B, the angled segments 134 can be angled by about 45 degrees relative to the common axis X1 of the stent 110. In other examples, the angled segments 134 can be angled by any angle such as about 30 degrees to about 90 degrees relative to the common axis X1 of the stent 110. In order to form the funnel shape, the angled segments 134 of the first and second elongated connectors 116a, 116b can be angled in different and/or opposite directions. For example, the first elongated connector 116a can be angled in a first direction, as shown by arrow A1 (in FIGS. 2A and 2B), and the angled segment 134 of the second elongated connector 116b can extend in a second direction shown by arrow A2 (in FIGS. 2A and 2B). An angle between the first direction A1 and the second direction A2 can be any non-zero angle (e.g., at least 60 degrees, at least 90 degrees, at least 120 degrees, etc.) depending, for example, upon dimensions of the rings 118a, 118b and/or other portions of the stent 110 as well as a degree to which the stent 110 has been expanded.

The elongated connectors 116a, 116b can also include bent portions 136 between the axially extending segments 132 of the elongated connectors 116a, 116b and the rings 118a, 118b. For example, the first elongated connector 116a can include a bent portion 136 extending between the first ring 118a and the elongated connector 116a and another bent portion 136 between the second ring 118b and the elongated connector 116a. The bent portions 136 of the first elongated connector 116a can both extend or bend in substantially a same direction, as shown by arrow A3 (in FIGS. 2A and 2B). The second elongated connector 116b can also include a bent portion 136 extending between the first ring 118a and the axially extending segment 134 of the second elongated connector 116b and another bent portion 136 extending between the adjacent or second ring 118b and another axially extending segment 134 of the second elongated connector 116b. The bent portions 136 of the second elongated connector 116b extend from the rings 118a, 118b in a second direction, shown by arrow A4 (in FIGS. 2A and 2B), which is different from, such as opposite from, the first direction of the bent portions 136 of the first elongated connector 116a.

The stent 110 can also include other connectors (not shown in FIGS. 2A and 2B), such as axial struts, or other connector members between rings 118 that vary in width, thickness, and/or shape compared to the elongated connectors 116a, 116b that exhibit the funnel shape. For example, such other connectors can be used to provide additional rigidity to certain portions of the stent 110 (e.g., in the rigid portion of the stent 110). Other connectors can also be added so that some portions of the stent 110 bend easily while other portions of the stent require a greater force to bend.

As shown in FIG. 2A, the stent 110 includes multiple similar or identical groups or pairs of the elongated connectors 116a, 116b that form multiple funnel shapes of the stent 110. The elongated connectors 116a, 116b are generally identical in shape and configuration forming the funnel shape having a narrow hinge point and a wide side. The elongated connectors 116a, 116b of this embodiment are symmetrical about a line parallel to the common axis X1 of the stent 110. Further, as shown in FIG. 2A, in this embodiment there are two groups or pairs of the elongated connectors 116a, 116b between each ring 118a and the immediately adjacent ring 118b, which are positioned on opposite sides of the rings 118a, 118b circumferentially spaced apart by about 180 degrees. For example, as shown in FIG. 2A, a first pair of the elongated connectors 116a, 116b extends between the first ring 118a and the second ring 118b at a 45 degree position and a second pair of the elongated connectors 116a, 116b extends between the first ring 118a and the second ring 118b at a 225 degree position.

However, while each funnel shape is generally similar in shape to other funnel shapes of the stent 110, the similar or identical funnel shapes can be arranged in various orientations and configurations throughout the flexible portion 112 of the stent 110 to impart different amounts of flexibility to different portions of the stent 110 and/or to cause some portions or regions of the stent 110 to flex or bend in any of a variety of desired directions along the length of the stent 110. In a simplest example, groups or pairs of the elongated connectors 116a, 116b can be generally axially aligned along an entire length or substantially an entire length of the flexible portion 112 of the stent 110. In such a configuration, the stent 110 may have a plurality of hinging axes that are parallel to one another and perpendicular to the common axis X1 such that the stent 110 may bend at one or more of these hinging axes, for example, so that the stent 110 exhibits a desired curvature (e.g., matching a natural curvature of the anatomy within which the stent 110 is to be deployed). In other examples, as shown in FIGS. 2A-4C, the elongated connectors 116a, 116b extending between different rings 118a, 118b, 118c, 118d of the stent 110 are not axially aligned as subsequent pairs of the elongated connectors 116a, 116b are circumferentially offset and/or are arranged in a more complex pattern.

More specifically, as shown in FIG. 2A, there are two funnel shapes formed by two groups or pairs of the elongated connectors 116a, 116b extending between the first ring 118a and the immediately adjacent or second ring 118b. The groups or pairs of the elongated connectors 116a, 116b are generally identical both including the first elongated connector 116a and the second elongated connector 116b. Further, the pairs or groups of the elongated connectors 116a, 116b are positioned on opposite sides of the stent 110. As shown in FIGS. 2A and 2B, the narrow hinge point or narrow side of the funnel shape of the elongated connectors 116a, 116b connects to the first ring 118a and the wide base or wide side of the funnel shape of the elongated connectors 116a, 116b connects to the second ring 118b.

The stent 110 also includes two groups or pairs of elongated connectors 116a, 116b extending from the second ring 118b to the third ring 118c. However, the elongated connectors 116a, 116b extending from the second ring 118b to the third ring 118c are circumferentially offset or not axially aligned with the elongated connectors 116a, 116b extending between the first ring 118a and the second ring 118b. Also, the elongated connectors 116a, 116b extending between the second ring 118b and the third ring 118c are rotated 180 degrees relative to the previous connectors 116a, 116b, such that the wide base or wide side of the funnel shape of the elongated connectors 116a, 116b is connected to the second ring 118b and the narrow hinge point or narrow side of the funnel shape of the elongated connectors 116a, 116b is connected to the third ring 118c. There are also two groups or pairs of elongated connectors 116 extending between the third ring 118c and the four ring 118d. The connectors 116a, 116b extending between the third ring 118c and the fourth ring 118d are in the same orientation and axially aligned with connectors 116a, 116b extending between the first ring and the second ring 118b. In this manner, the stent 110 as illustrated in FIG. 2A alternates the positioning and orientation of the elongated connectors 116a, 116b when moving from a pair of the rings 118 to the next pair of the rings 118, the pair and the next pair of the rings having a common one of the rings 118.

The configuration and orientation of the funnel shapes and/or the elongated connectors 116a, 116b shown in FIGS. 2A and 2B is not intended to be limiting of the present disclosure, and other configurations and orientations for funnel shapes or the elongated connectors 116a, 116b may be determined by those skilled in the art to produce stents 110 that bend or flex in certain desired directions for particular applications. Some such alternative configurations and orientations for the elongated connectors 116a, 116b are shown in FIGS. 3A, 3B, 4A, 4B and 4C, and are described in further detail herein. Other configurations and orientations for the elongated connectors 116a, 116b may also be implemented within the scope of the present disclosure.

### Multi-directional hinging stent with funneled hinges forming a spiral

FIGS. 3A and 3B show other examples of a flexible portion 112 of a stent 110 comprising elongated connectors 116a, 116b arranged for each pair to exhibit a funnel shape. As in FIGS. 2A and 2B, the elongated connectors 116a, 116b shaped and positioned according to the present disclosure provides a flexible stent to allow bending or hinging requiring a reduced amount of force while preventing kinking, but also has significant column strength. Therefore, the stent 110 can be used for endovascular applications where flexibility is required, but where resistance to longitudinal deformation is also important. As shown in FIGS. 3A and 3B, the elongated connectors 116a, 116b of the flexible portions 112 are arranged to form spirals extending axially along a length of the flexible portion 112 of the stent 110, as shown by lines L2, L3, and L4 in FIGS. 3A and 3B. As shown in FIGS. 3A and 3B, the funnel shape of the elongated connectors 116a, 116b can be arranged such that the wide base faces toward the distal end 104 of the stent 110 as in FIG. 3A, faces toward the proximal end 102 of the stent 110 as in FIG. 3B, or in any combination of proximal and distal facing funnel shapes. In other examples, connection points 128, 130 between the elongated connectors 116a, 116b and the rings 118 can be arranged in any order so as to obtain the desired stent flexibility for the intended application.

More specifically, as shown in FIG. 3A, the flexible portion 112 of the stent 110 comprises eight expandable rings, including the proximal-most or first ring 118a, the second ring 118b, the third ring 118c, the fourth ring 118d, and additional rings 118, aligned in series along the common axis X1 of the stent 110. The rings 118 comprise the multiple linear segments 122 connected together end-to-end forming peaks 124 and valleys 126 of the rings 118, as in previous examples. Each ring 118 in FIG. 3A includes eight peaks 124 and eight valleys 126, though as described above, any number of rings 118, linear segments 122, peaks 124, and valleys 126 may be utilized for the stent 110. The rings 118 are arranged in a peak-to-peak configuration.

The flexible portion 112 in FIG. 3A further comprises two groups or pairs of elongated connectors 116a, 116b between each ring, such as the first ring 118a, and the immediately adjacent ring, such as the second ring 118b, where each pair of elongated connectors 116a, 116b have a funnel shape with the narrow hinge point of each funnel shape being closer to the proximal end 102 and the wide base being closer to the distal end 104. As in previous examples, the groups or pairs of the elongated connectors 116a, 116b between each ring 118a and the immediately adjacent ring 118b are on opposite sides of the stent 110 separated by about 180 degrees. Further, the elongated connectors 116a, 116b of each group or pair are symmetrical about a line that is parallel to the common axis X1 of the stent 110, as in previous examples. As shown in FIG. 3A, there are eight different positions where the elongated connectors 116a, 116b between a ring 118 and an immediately adjacent ring 118 could be positioned as shown by Positions 1-8 illustrated in FIG. 3A. Stents 110 with fewer peaks 124 and valleys 126 per ring 118 element will have fewer potential connection locations for the elongated connectors 116a, 116b. Stents 110 including the rings 118 with more than eight peaks 124 and eight valleys 126 will have more potential connection locations for the elongated connectors 116a, 116b.

As previously described, the elongated connectors 116a, 116b in FIG. 3A are arranged to form the spirals (shown by lines L2, L3, L4) extending along the length of the flexible portion 112. In order to provide the spiral configuration, as shown in FIG. 3A, the elongated connectors 116a are axially offset (e.g., from one ring 118 to another ring 118) and circumferentially offset (e.g., rotating about a circumference of the stent 110) from adjacent ones of the elongated connectors 116a. When considering different elongated connectors 116a, a first elongated connector 116a with a connection point 128 closer the proximal end 102 on the first ring 118a is axially and circumferentially offset to a second elongated connector 116a with a connection point 128 closer the proximal end 102 on the second ring 118b. The circumferential offset between these two connection points 128 of the elongated connectors 116a (e.g., if the rings 118a, 118b were transposed over one another) is by a ring length comprising two linear segments 122, such that a connection point 128 for the first elongated connector 116a appears to move up (e.g., toward a top of the stent 110) and over from a connection point 128 from the second elongated connector 116a, with the same configuration applying to each of the elongated connectors 116b and their connection points 130. When considering the same elongated connector 116a, as shown in FIG. 3A, in this spiral configuration, a connection point 128 for a first elongated connector 116a on a particular ring 118a that is closer to the proximal end 102 is axially and circumferentially offset to a connection point 128 for the first elongated connector 116a on a particular ring 118b. The circumferential offset between these two connection points 128 of the same elongated connector 116a (e.g., if the rings 118a, 118b were transposed over one another) is by a ring length comprising one linear segment 122. Similarly, in this spiral configuration, the connection points 130 for the elongated connector 116b compared to another one of the elongated connectors 116b or within a single one of the elongated connectors 116b are axially and circumferentially offset. For example, the circumferential offset if transposed would be a ring length comprising two linear segments 122 when considering the connection points 130 for two different ones of the elongated connectors 116b and the circumferential offset if transposed would be a ring length comprising one linear segment 122 when considering the connection points 130 for a single one of the elongated connectors 116b). Accordingly, the elongated connectors 116a, 116b between each of the rings 118a, 118b are equidistantly spaced apart about a circumference of the rings 118 (e.g., 180 degrees apart) and appear to spiral about the stent 110, as shown by lines L2, L3, and L4 in FIG. 3A.

FIG. 3B is a mirror image of FIG. 3A about a plane perpendicular to the common axis X1 showing the elongated connectors 116a, 116b in another orientation, in which elongated connectors 116a, 116b spiral in a downward direction, as shown by lines L2, L3, and L4 in FIG. 3B. More specifically, as shown in FIG. 3B, the connection points 128, 130 of the elongated connectors 116a, 116b extending from any one of the rings 118, 118a, 118b, 118c, 118d, in a proximal direction (i.e., towards a proximal end 102 of the stent 110) are axially and circumferentially offset from the connection points 128, 130 of the elongated connectors 116a, 116b extending from the particular ring 118 in a distal direction (i.e., towards the distal end 104 of the stent 110), such that each of the elongated connectors 116a, 116b appears to move down (e.g., toward a bottom of the stent 110) one step and over from the previous elongated connectors 116a, 116b.

### Multi-directional hinging stent with funneled hinges with increased or uneven spacing

FIGS. 4A-4C show additional examples of the stents 110 including the elongated connectors 116a, 116b that exhibit the funnel shape. Similar to previous examples, the stents 110 of FIGS. 4A-4C include two groups or pairs of the elongated connectors 116a, 116b extending between each ring 118, such as a first ring 118a, and the immediately adjacent ring, such as a second ring 118b. Also, as in previous examples, the pairs or groups of the elongated connectors 116a, 116b are positioned on opposite sides of the rings 118a, 118b separated by about 180 degrees about the circumference of the rings 118a, 118b. However, the flexible portion 112 of FIGS. 4A-4C differs from previous examples, in spacing between the elongated connectors 116a, 116b on the ring 118a and on the rings 118b, 118c, 118d.

More specifically, as shown in FIG. 4A, the flexible portion 112 can include seven rings, such as the proximal-most or first ring 118a, the second ring 118b, the third ring 118c, the fourth ring 118d, and additional rings 118, all of which are aligned in series. However, the number of the rings 118a-118d is not intended to be limiting and, in other examples, the flexible portion 112 of the stent 110 can include any number of rings, e.g., fewer than seven rings or more than seven rings. For example, the flexible portion 112 could include six, eight, nine, twelve, or more rings 118. As shown in FIG. 4A, there are two groups or pairs of the elongated connectors 116a, 116b extending between each ring 118a and an immediately adjacent ring 118b. Each group or pair of the elongated connectors 116a, 116b forms a funnel shape, and each pair of the elongated connectors 116a, 116b in the stent 110 of FIG. 4A face in the same direction. For example, each funnel shape of the elongated connectors 116a, 116b has the narrow hinge point closer to the proximal end 102 while the wide base is closer to the distal end 104.

More specifically, as shown in FIG. 4A, the flexible portion 112 includes two groups or pairs of the elongated connectors 116a, 116b extending between the first ring 118a and the second ring 118b. The flexible portion 112 further includes two groups or pairs of the elongated connectors 116a, 116b extending between the second ring 118b and the adjacent third ring 118c. The connectors 116a, 116b between the second ring 118b and the third ring 118c are axially and circumferentially offset from the connectors 116a, 116b between the first ring 118a and the second ring 118b by a ring length comprising four linear segments 122, when comparing like connection points 128, 130 on like elongated connectors 116a, 116b. The stent 110 also includes the elongated connectors 116a, 116b extending from the third ring 118c to a fourth ring 118d. These elongated connectors 116a, 116b between the third ring 118c and the fourth ring 118d are axially aligned with the elongated connectors 116a, 116b between the first ring 118a and the second ring 118b. Also, the elongated connectors 116a, 116b between the third ring 118c and the fourth ring 118d are axially and circumferentially offset from the elongated connectors 116a, 116b extending between the second ring 118b and the third ring 118c by a ring length comprising four linear segments 122, when comparing like connection points 128, 130 on like elongated connectors 116a, 116b. The pattern of spaced-apart and axially aligned elongated connectors 116a, 116b repeats several times along a length of the flexible portion 112 of the stent 110. For example, as shown in FIG. 4A, the flexible portion 112 includes seven rings, with the pattern of spaced-part and axially aligned connectors repeating twice. This configuration of the pairs of the elongated connectors 116a, 116b also results in each ring 118 connecting to both a narrow hinge point and a wide base. The connections to the narrow hinge point and the wide base alternate progressively around the ring 118 (e.g., wide-narrow-wide-narrow).

FIG. 4B shows another exemplary flexible portion 112 of the stent 110 including the elongated connectors 116a, 116b having the funnel shape of the present disclosure. The flexible portion 112 in FIG. 4B includes seven rings, such as a proximal-most or first ring 118a, a second ring 118b, a third ring 118c, a fourth ring 118d, and additional rings 118, which are aligned in series along the common axis X1. The elongated connectors 116a, 116b of FIG. 4B are arranged in a variety of configurations and orientations. For example, the funnel shapes of the elongated connectors 116a, 116b extending between the first ring 118a and the second ring 118b of the stent 110 have the wide base closer to the proximal end 102 and the narrow hinge point closer to the distal end 104.

By contrast, the funnel shapes of the elongated connectors 116a, 116b extending between the second ring 118b and the third ring 118c have the narrow hinge point closer to the proximal end 102 and the wide base closer to the distal end 104. Also, the elongated connectors 116a, 116b that extend proximally from the second ring 118b are circumferentially offset from the elongated connectors 116a, 116b extending distally from the second ring 118b by a ring length comprising multiple linear segments 122, such as by two or more linear segments 122 (e.g., four linear segments 122 as shown in FIG. 4B). In FIG. 4B, the elongated connectors 116a, 116b extending between the third ring 118c and the fourth ring 118d are in the same orientation and position as the elongated connectors 116a, 116b extending between the first ring 118a and the second ring 118b. The elongated connectors 116a, 116b extending between the fourth ring 118d and the next distally adjacent one of the rings 118 are in the same orientation and position as the elongated connectors 116a, 116b extending between the second ring 118b and the third ring 118c. Also, the pattern of spaced-apart and axially aligned elongated connectors 116a, 116b repeats several times along a length of the flexible portion 112 of the stent 110. For example, as shown in FIG. 4B, the flexible portion 112 includes seven rings, with the pattern of spaced-part and axially aligned connectors repeating twice. This configuration of the pairs of the elongated connectors 116a, 116b also results in each ring 118 connecting to either only a narrow hinge point or only a wide base (e.g., wide-wide-wide-wide or narrow-narrow-narrow-narrow).

FIG. 4C shows a further exemplary flexible portion 112 of the stent 110 including the elongated connectors 116a, 116b having the funnel shape of the present disclosure. The flexible portion 112 in FIG. 4C includes seven rings, such as a proximal-most or first ring 118a, a second ring 118b, a third ring 118c, a fourth ring 118d, and additional rings 118, which are aligned in series along the common axis X1. The elongated connectors 116a, 116b of FIG. 4C are arranged in a yet another manner of configurations and orientations. Specifically, FIG. 4A shows when the funnel shapes of the elongated connectors 116a, 116b all face the same direction. FIG. 4B shows when the funnel shapes of the elongated connectors 116a, 116b alternate between a first direction and a second, opposite direction for progressive pairs of adjacent ones of the rings 118. As described below, FIG. 4C shows when the funnel shapes of the elongated connectors 116a, 116b alternate between a first direction and a second, opposite direction for a given pair of adjacent ones of the rings 118.

For example, for the elongated connectors 116a, 116b extending between the first ring 118a and the second ring 118b of the stent 110, a first pair of the elongated connectors 116a, 116b has the wide base closer to the proximal end 102 and the narrow hinge point closer to the distal end 104. A second pair of the elongated connectors 116a, 116b that also extends between the first ring 118a and the second ring 118b of the stent 110 has the narrow hinge point closer to the proximal end 102 and the wide base closer to the distal end 104. Also, the elongated connectors 116a, 116b that extend proximally from the second ring 118b are circumferentially offset from the elongated connectors 116a, 116b extending distally from the second ring 118b by a ring length comprising multiple linear segments 122, such as by two or more linear segments 122 (e.g., two linear segments 122 as shown in FIG. 4C). For each pair of adjacent ones of the rings 118, the elongated connectors 116a, 116b may be grouped such that a first pair of the elongated connectors 116a, 116b has a first direction and a second pair of the elongated connectors 116a, 116b has a second, opposite direction, with the first pair of the elongated connectors 116a, 116b being circumferentially offset from the second pair of the elongated connectors 116a, 116b by, for example, 180 degrees. The pairs of the elongated connectors 116a, 116b may be oriented such that there is an axial alignment. The axial alignment of the elongated connectors 116a, 116b alternate between the first and second directions of the funnel shape. The pairs of elongated connectors 116a, 116b also appear to spiral about the stent 110, in a manner substantially similar to the spiral shown via lines L2, L3, and L4 in FIGS. 3A and 3B. Specifically, the pairs of elongated connectors 116a, 116b having a funnel shape that faces the same direction exhibit the spiral when connecting these like directional funnel shapes progressively along a length of the stent 110. This configuration of the pairs of the elongated connectors 116a, 116b also results in each ring 118 connecting to both a narrow hinge point and a wide base. The connections to the narrow hinge point and the wide base are paired progressively around the ring 118 (e.g., wide-wide-narrow-narrow).

As previously described, other arrangements of the elongated connectors 116a, 116b may also be apparent to those skilled in the art including arrangements where the groups or pairs of the elongated connectors 116a, 116b are aligned in simple repeating patterns or other arrangements where the elongated connectors 116a, 116b are not axially or circumferentially aligned and/or are arranged in an uneven or more random distribution that does not follow an identifiable pattern. For example, the elongated connectors 116a, 116b that have the funnel shape can be positioned based on trial and error or finite element analysis studies in specific positions on the rings 118 to achieve desired flexibility and/or column strength. In such cases, the elongated connectors 116a, 116b may not be arranged in repeating patterns, such as the patterns shown in FIGS. 2A, 2B, 3A, 3B, 4A, and 4B.

### Deployment methods for multi-directional hinging stents

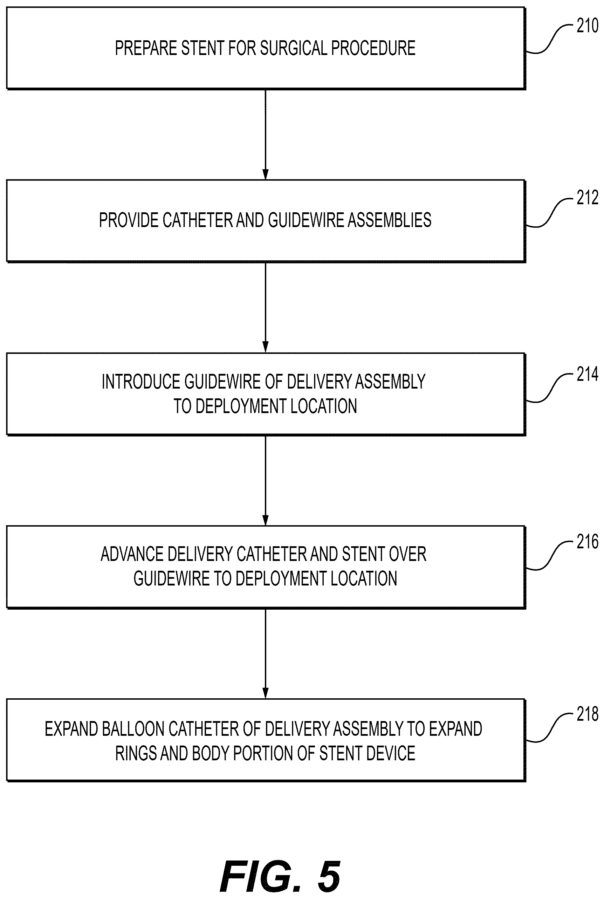

FIG. 5 is a flow chart showing a method for deploying a stent 10, 110 including a multi-directional hinging mechanism. The deployment method can be applicable to any of the stents 10, 110 embodiments of this disclosure. As shown at step 210 of the method, a selected stent 10, 110 is prepared for surgery by removing it from its packaging and removing a protective sheath that covers the stent 10 during storage. The stent 10 is initially provided in a compressed configuration, such as crimped over a balloon expandable catheter. As previously described, the stents 10, 110 are configured to bend or hinge easily in one or more directions based on positioning of the elongated connectors 16, 116a, 116b and/or other connections between rings 18, 118 of the stent 10, 110. Stents 10, 110 can be selected for particular surgical procedures and target deployment sites based on how the stent 10, 110 is required to bend or hinge when deployed as well as dimensions such as the length and diameter of the stent as would be understood by those skilled in the art. For example, a multi-directional hinging stent 10, 110 that bends easily in more than one direction and which can be bent around a sharp corner without kinking may be useful for surgical procedures in which the stent is positioned within a branched artery or where the stent 10, 110 extends through an opening from one body passageway to another. Multi-directional hinging stents 10, 110 may also be selected for use in deployment locations where the stent 10, 110 is likely to be exposed to multiple different bending forces and/or is likely to be bent or twisted in multiple directions along its length. Additional uses for stents that bend easily in multiple directions will also be easily determined by those skilled in the art.

At step 212 of the method, a delivery assembly comprising a catheter or sheath and a guidewire for advancing the stent 10, 110 through vasculature of a patient to a deployment location is provided. The deployment location can be any desired position within the vasculature of the patient. For example, the stent 10, 110 can be deployed in a vessel or artery. In some examples, the stent 10, 110 is deployed in conjunction with an endograft. As previously described, a stent with specific hinging and bending characteristics can be selected for a particular desired surgical procedure and deployment location.

The stent 10, 110 is crimped over the balloon catheter and can be inserted in a delivery catheter. In order to deploy the stent 10, 110, at step 214 of the method, the guidewire is introduced through the vasculature to the desired deployment location. Once the guidewire is in place, at step 216 of the method, the delivery catheter and the balloon catheter with the stent device mounted thereto are advanced to the deployment location over the guidewire.

At step 218 of the method, once the stent 10, 110 is at the desired deployment location, the balloon catheter is expanded. Radial outward expansion of the expandable portion of the balloon catheter causes the expandable rings 18, 118 of the stent 10, 110 to expand radially outward, as described previously. Following expansion, the guidewire can be removed allowing the stent 10, 110 to adopt a curved, bent, or hinged configuration, as required by the surgical procedure and deployment site.

### EXAMPLES

The following examples are presented to demonstrate general principles of embodiments in accordance with this disclosure. This disclosure, and any claimed embodiments, should not be considered as limited to the specific examples presented.

### Hypothetical Example 1

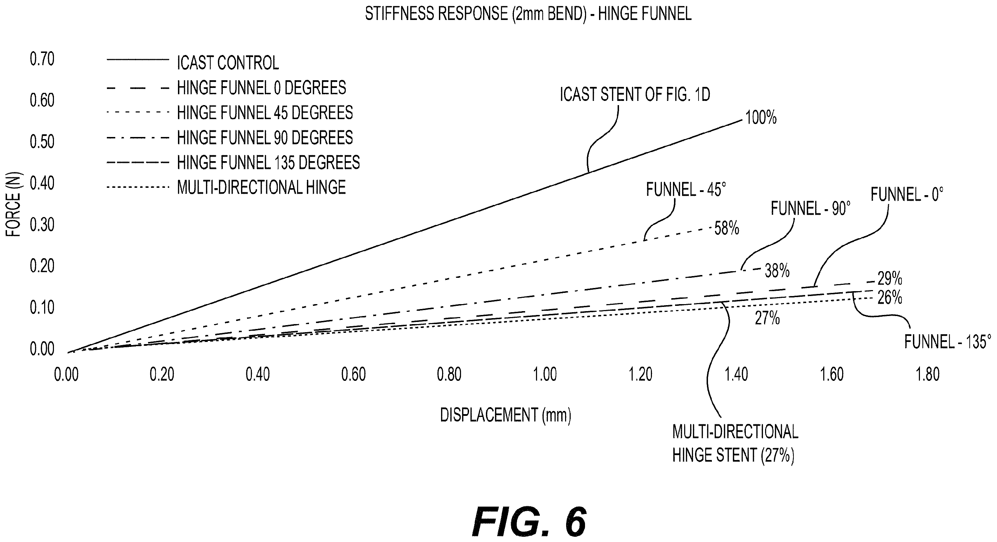

As previously described, the multi-directional hinging stent 10 shown in FIGS. 1A-1D and the stent 110 including the elongated connectors 116a, 116b exhibiting the funnel shape in FIGS. 2A-4C are provided to increase the flexibility of the stent 10, 110 as compared to rigid conventional stents (e.g., conventional stents with the rigid portion extending throughout) allowing the stents 10, 110 to flex or bend easily in multiple directions at selected locations. That is, the stent 110 may bend or hinge about a hinging axis that is created by the positioning of the elongated connectors 116a, 116b with a reduced amount of force as compared to bending or hinging the stent 10, 110 at locations other than about the hinging axis. FIG. 6 is a graph comparing a bending stiffness response of multi-directional hinging stents including features or the present disclosure to other control or conventional stents, such as the stent 10 and a conventional stent including axial struts positioned about the circumference of the stent, such as in a manner substantially similar to the rigid portion shown in FIG. 1D.

Specifically, FIG. 6 provides a graphical representation of the forces that are required to bend an iCast stent, a multi-directional hinging stent, and a stent with the elongated connectors 116a, 116b exhibiting the funnel shape by a distance of 2 mm as calculated by finite element analysis (FEA). The bending of the distance of 2mm may be in the direction that requires the reduced amount of force as defined by the hinging axis created by the elongated connectors 16 and the elongated connectors 116a, 116b for the stents 10, 110, respectively. FIG. 6 demonstrates that by altering the design of a conventional iCAST stent having four connectors, which may have the same configuration as the rigid portion 14 of the stent 10 as seen in FIG. 1D, a significant reduction in bending force is realized. As shown in FIG. 6, when a multi-directional hinging stent is bent using FEA hinge analysis the force is 27% of the force required to bend the control stent or a 73% reduction in bending force. When the stent including the elongated connectors 116a, 116b oriented to exhibit the funnel shape is bent in the direction of the hinge (e.g., 0 degrees) the force is 29% of the force required to bend the control stent or a 71% reduction in bending force. When the stent is bent 45 degrees to the direction of the hinge, the force is 58% of the force required to bend the control stent or a 42% reduction in bending force. When the stent is bent 90 degrees to the direction of the hinge the force is 38% of the force required to bend the control stent or a 62% reduction in bending force. When the stent is bent 135 degrees to the direction of the hinge the force is 26% of the force required to bend the control stent or a 74% reduction in bending force. The FEA data presented in FIG. 6 demonstrates that a stent including funneled hinges as described in these embodiments results is a stent with a much lower bending force required and a far more flexible stent. It is noted that the relative amounts of force measured in the graph of FIG. 6 are based on the elongated connectors used in the various stents being of a common material. As such, the relative amounts of force may change based on different materials used for the connectors. However, a relative degree by which the force is reduced may be similar based on using the stent design incorporating the elongated connectors described in the present disclosure, with particular emphasis on the elongated connectors 116a, 116b. For example, the ratio of forces required to bend the stents 10, 110 may be substantially similar, independent of the material being used. In this manner, the stent design may also contribute to the bending stiffness along with the material.

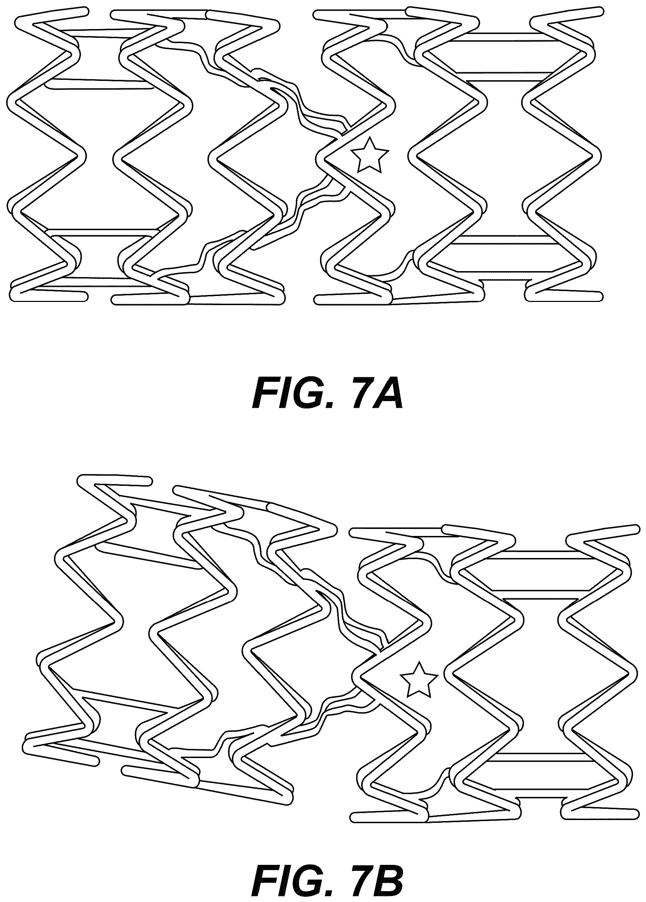

FIGS. 7A and 7B are computer generated models (FEA models) showing a bend of a flexible portion 112 of the multi-directional hinging stent 110 including the elongated connectors 116a, 116b exhibiting the funnel shape where two pairs of the elongated connectors 116a, 116b are positioned between the same rings 118 and circumferentially offset by 180 degrees. Specifically, the stent 110 is shown in a straight orientation or in a linear state in FIG. 7A with the elongated connectors 116a, 116b in a non-deformed or rest position. As shown in FIG. 7B, the stent 110 is in a bent orientation, as modeled using FEA, or in an angled state with the elongated connectors 116a, 116b in a deformed or stressed position. It is evident from this illustration that the elongated connectors 116a, 116b provide the narrow hinge point, shown by the star (in FIG. 7B), which is adjacent and proximate to the narrow side of the funnel shape of both pairs of the elongated connectors 116a, 116b, where the stent can bend more easily by requiring a reduced amount of force to bend the stent 110, yielding an extremely flexible stent. The star also represents a point through which the hinging axis extends through the stent 110. Accordingly, in FIGS. 7A and 7B, the hinging axis created by the two pairs of the elongated connectors 116a, 116b may extend through the star, perpendicular to a plane of the drawing. The angled state of the stent 110 in FIG. 7B also illustrates how the elongated connectors 116a, 116b prevent kinking to maintain the lumen of the stent 110 with minimal to no change. Those skilled in the art will also appreciate that the hinging axis may be used singularly or in combination with one or more other hinging axes. For example, when the stent 110 hinges about a single hinging axis, the stent 110 may be bent or angled. In this manner, the stent 110 may accommodate sharp bends. In another example, when the stent 110 hinges about multiple hinging axes where the hinging axes are parallel and co-planar, the stent 110 may be rounded or exhibit a curve.

The example described above with regard to the stent 110 illustrated in FIGS. 7A-7B is directed to the exemplary embodiments where the funnel shapes of the pairs of the elongated connectors 116a, 116b at a given pair of the rings 118 are in the same direction (e.g., the narrow hinge point is closer to the proximal end 102 and the wide base is closer to the distal end 104). For example, the stent 110 having this configuration is shown in FIGS. 2A, 3A, 3B, 4A, and 4B. As described above, the resulting hinging axis from this configuration is orthogonal to the common axis X1. However, the orthogonal nature of the hinging axis is only for illustrative purposes and the exemplary embodiments may also create a non-orthogonal hinging axis relative to the common axis X1. For example, the stent 110 may also have a configuration where the funnel shape of the pairs of the elongated connectors 116a, 116b at a given pair of the rings 118 face different directions (e.g., the funnel shape of a first pair of the elongated connectors 116a, 116b have the narrow hinge point closer to the proximal end 102 and the wide base closer to the distal end 104 while the funnel shape of a second pair of the elongated connectors 116a, 116b circumferentially offset by 180 degrees to the first pair has the narrow hinge point closer to the distal end 104 and the wide base closer to the proximal end 102). The stent 110 having this configuration is shown in FIG. 4C. With the hinging axis extending based on the narrow hinge points, the hinging axis may be at a non-perpendicular angle to the common axis. The length of the connectors 116a, 116b or a distance between the rings 118 may define the non-perpendicular angle by which the hinging axis is relative to the common axis X1. Again, it is noted that the above description and the directions of the funnel shapes are for the pairs of the elongated connectors 116a, 116b at a given pair of the rings 118. In a different perspective, when considering the directions of the funnel shapes from one pair of rings 118 to an adjacent pair of rings 118 in the longitudinal direction of the stent 110, the directions of the funnel shapes may be the same (e.g., FIG. 4A), may alternate (e.g., FIG. 4B), or may form spirals (e.g., FIG. 4C).

### Hypothetical Example 2

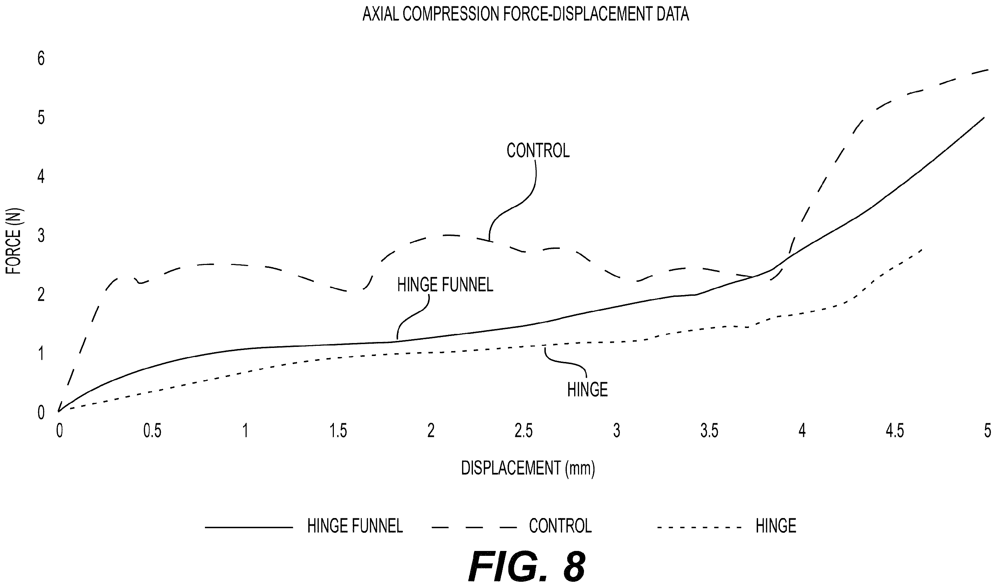

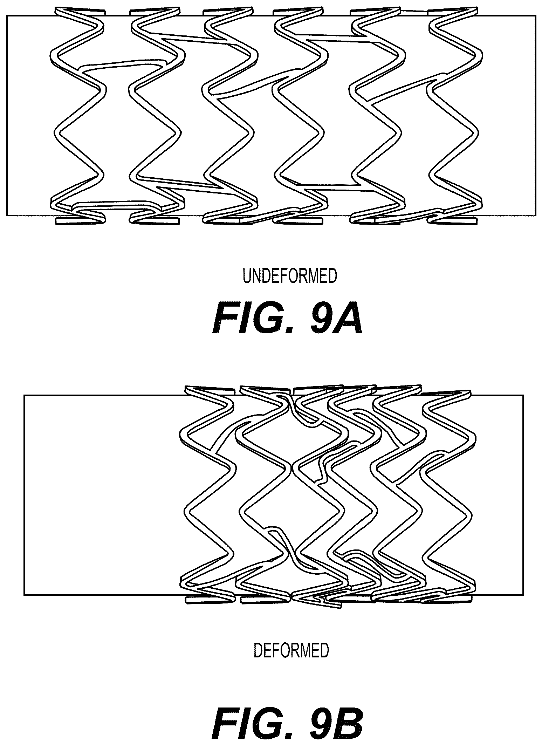

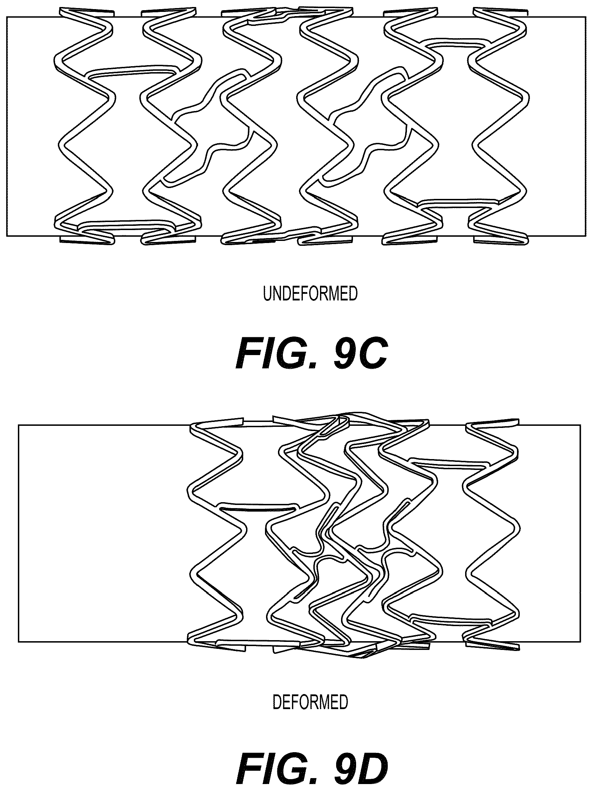

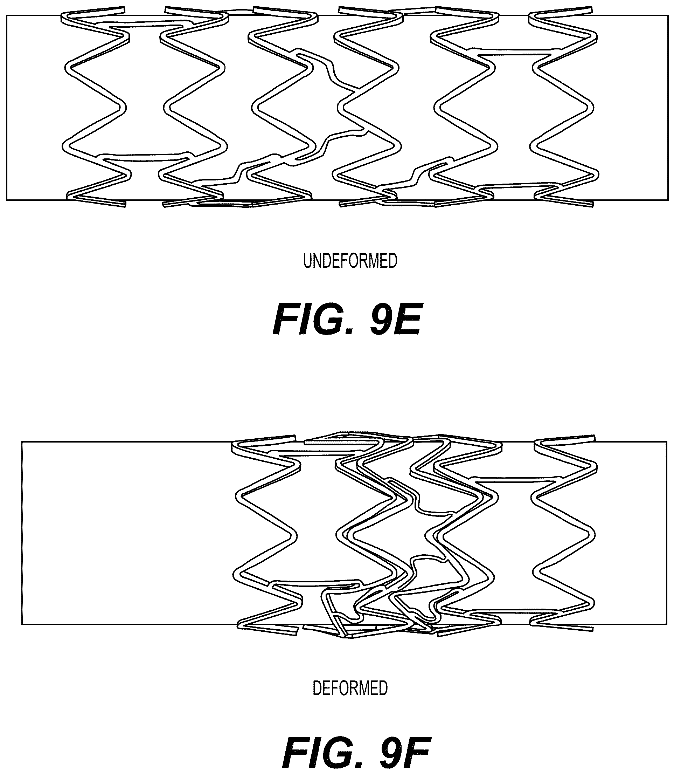

Finite Element Analysis (FEA) was also used to measure the behavior of a multi-directional hinging stent 10, a control iCast stent, and a stent 110 with the elongated connectors 116a, 116b exhibiting the funnel shape in terms of axial compression force or column strength. Results of the FEA analysis for axial compression force are shown in FIG. 8. Computer generated images showing the different stents in a non-deformed or rest configuration and a deformed or stressed configuration where the stents are bent are shown in FIGS. 9A-9F. Specifically, FIGS. 9A-9B show a stent design with isolated single elongated connectors positioned between rings. FIGS. 9C-D show a stent design with paired elongated connectors positioned between rings where the elongated connectors of a pair have the same shape and orientation. FIGS. 9E-F show a stent design corresponding to the stent 110 with paired elongated connectors positioned between rings where the elongated connectors of a pair exhibit the funnel shape.

In order to produce the displacement and force values shown in FIG. 8, the FEA models for the different stents were expanded to 8 mm and then subjected to an axial compression load. The force to compress the stent as a function of displacement is plotted in FIG. 8. As shown in FIG. 8, the control stent being the iCAST stent shown in FIGS. 9A-B having isolated connectors (e.g., corresponding to only the rigid portion 14 shown in FIG. 1D) demonstrated the highest column strength. However, as previously described, the control stent also had the least amount of flexibility. The multi-directional hinging stent including the elongated connectors 16 such as those illustrated in FIGS. 1A-D that are in a paired configuration as shown in FIGS. 9C-D had the lowest overall axial compression force but had very high flexibility. The stent 110 including the elongated connectors 116a, 116b exhibiting the funnel shape and shown in FIGS. 9E-F maintained high flexibility similar to the multi-directional hinging stent, but had an increase in axial compression force compared to the stent of FIGS. 9C-D. As previously described, it is believed that this improvement in column strength, while maintaining flexibility, provides benefits to the manufacturing process as well as the device performance. For example, the process to manufacture the stent 110 may be improved with increased efficiency as the processing operation steps may be performed with greater success due to the increased column strength that decreases errors and damage that may be due to the processing operation steps, where the errors and damage may ultimately cause the stent to be discarded.

### Hypothetical example 3

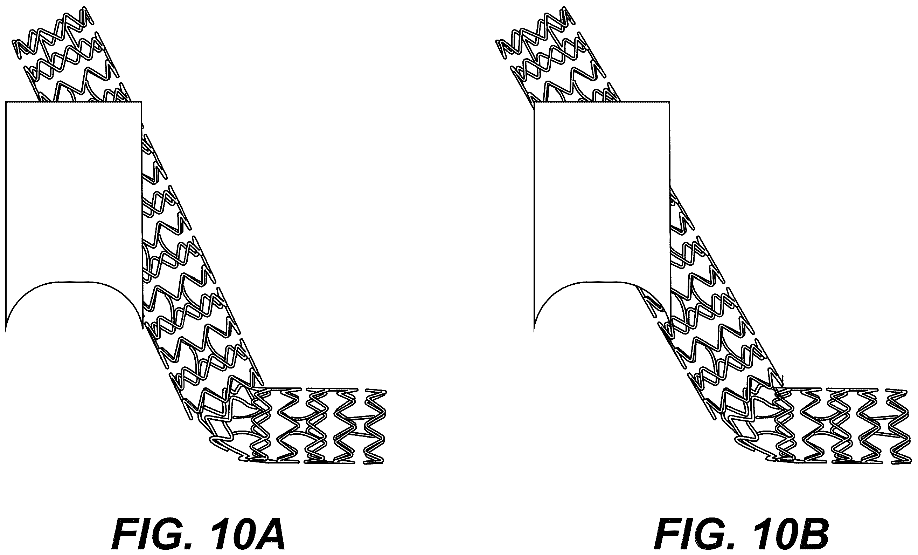

FIGS. 10A and 10B are visual representations created using finite element analysis (FEA) to approximate stent behavior during a cantilever test comparing a conventional control stent (FIG. 10A), such as an iCast stent, and a multi-directional stent including the elongated connectors 116a, 116b exhibiting the funnel shape (FIG. 10B), similar to the stent 110 shown in FIG. 2A.

The conventional stent (FIG. 10A) is a conventional stainless steel stent including four elongated connectors between each ring and adjacent rings positioned at 0 degrees, 90 degrees, 180 degrees, and 270 degrees, in a manner substantially similar to the rigid portion 14 as shown in FIG. 1D. The stent comprising the elongated connectors 116a, 116b (FIG. 10B) includes the two groups or pairs of the elongated connectors 116a, 116b between each ring 118 that exhibit the funnel shape (e.g., as shown in FIG. 2A). It is noted that the stents in FIGS. 10A and 10B include the same ring structure and configuration, and differ only in positioning and type of the elongated connectors between the rings.

To create the FEA representations, the stent designs were modeled when expanded to 8 mm diameter and then subjected to a cantilever simulation to a distance of 19 mm. FIG. 10A demonstrates that when subjected to this cantilever force, the conventional stent begins to kink to a point where the lumen diameter and cross-sectional area are decreased by a non-trivial amount.

When a flexible portion 112 of the stent 110 in FIG. 10B including the elongated connectors 116a, 116b exhibiting the funnel shape is subjected to the same cantilever force, the bending transition is smooth and without the presence of a kink such that the lumen diameter and cross-sectional area are not or minimally affected. These visual representations show that the addition of the multi-directional hinging mechanism provided by the stent utilizing the elongated connectors 116a, 116b allows the stent to create a smooth curve or sharp bend that allows the stent to conform to vessels or branches in any direction and in various angles.

Although various non-limiting embodiments of the invention have been described in detail for the purpose of illustration based on what is currently considered to be the most practical and preferred aspects, it is to be understood that such detail is solely for that purpose and that the invention is not limited to the disclosed aspects, but, on the contrary, is intended to cover modifications and equivalent arrangements that are within the spirit and scope of the appended claims. For example, it is to be understood that the present disclosure contemplates that, to the extent possible, one or more features of any aspect can be combined with one or more features of any other aspect.

## Claims

1. An expandable multi-directional hinging stent configured to radially expand from a compressed configuration to an expanded configuration, the expandable stent comprising:
a plurality of radially expandable rings aligned in series along a common axis and defining a common lumen of the expandable stent extending through the rings; and
a plurality of elongated connectors extending between adjacent ones of the rings, wherein a first one of the elongated connectors and a second one of the elongated connectors are connected to and extend between a first one of the rings and an immediately adjacent second one of the rings,
wherein a shortest distance about a circumference of the expandable stent between a connection point between the first connector and the first ring and a connection point between the second connector and the first ring is less than a shortest distance about a circumference of the expandable stent between a connection point between the first connector and the second ring and the second connector and the second ring.

2. The expandable stent of claim 1, wherein each of the rings comprises multiple linear segments connected together end-to-end forming peaks and valleys around a circumference of the ring,
wherein the connection point between the first connector and the first ring and the connection point between the second connector and the first ring are on adjacent linear segments separated from one another by a valley, and/or wherein the connection point between the first connector and the second ring and the connection point between the second connector and the second ring are on non-adjacent linear segments, and
wherein the non-adjacent linear segments of the second ring are separated from one another by a ring length comprising at least two complete linear segments connected through a valley of the second ring.

3. The expandable stent of claim 1, wherein each of the elongated connectors comprises two axially extending segments and an angled segment between the two axially extending segments angled relative to the common axis of the expandable stent, wherein the angled segment of the first connector is angled in a first direction relative to the common axis of the expandable stent and the angled segment of the second connector is angled in a second direction relative to the common axis of the expandable stent, the second direction being different from the first direction.

4. The expandable stent of claim 1, wherein the elongated connectors are arranged along a spiral extending axially through the rings.

5. The expandable stent of claim 1, wherein the first connector is symmetrical to the second connector about a line parallel to the common axis of the expandable stent.

6. The expandable stent of claim 1, wherein the first connector and the second connector form a first group, wherein the elongated connectors further comprise a second group comprising a third connector and a fourth connector extending between the first ring and the second ring.

7. The expandable stent of claim 8, wherein the first group and the second group are circumferentially offset about the first ring and the second adjacent ring such that a hinging axis is created that extends through the expandable stent based on a positioning of the first group and the second group, the expandable stent configured to hinge about the hinging axis between the first ring and the second ring.

8. The expandable stent of claim 9, wherein the first group and the second group are circumferentially offset from one another by 180 degrees.

9. The expandable stent of claim 9, wherein the expandable stent is configured to hinge between the first and second rings about the hinging axis with a first amount of force reduced relative to a second amount of force required to hinge the expandable stent between the first and second rings about an axis other than the hinging axis.

10. The expandable stent of claim 8, wherein the first connector and the second connector are paired to exhibit a funnel shape, the funnel shape being oriented in one of a first direction with a narrow hinge point closer to a proximal end of the expandable stent and a wide base closer to a distal end of the expandable stent or a second direction with the narrow hinge point closer to the distal end of the expandable stent and the wide base closer to the proximal end of the expandable stent.

11. The expandable stent of claim 13, wherein, between the first ring and the second ring, the funnel shape of the first group and the second group is one of both facing the first direction, both facing the second direction, or one facing the first direction and the other facing the second direction.

12. The expandable stent of claim 13, wherein, at progressive pairs of the radially expandable rings, the funnel shape of the first group and the second group is facing one of the first or second direction or alternates between the first and second directions.

13. The expandable stent of claim 1, wherein the rings comprise:
the first ring,
the second ring, and
a third radially expandable ring,
wherein the first ring, the second ring, and the third ring are aligned in series along the common axis of the expandable stent, and
wherein the elongated connectors comprise a third connector and a fourth connector axially offset from the first connector and the second connector the third connector and the fourth connector extending between the second ring and the third ring.

14. The expandable stent of claim 16, wherein the first connector and the second connector are circumferentially offset from the third connector and the fourth connector by no more than 180 degrees.

15. The expandable stent of claim 16, wherein the expandable stent is configured to bend about a hinging axis between the first ring and the second ring with a first amount of force in a first direction and to bend about a further hinging axis created by the third and fourth connectors between the second ring and the third ring with the first amount of force in a second direction different than the first direction.
